# EUROPEAN PATENT APPLICATION

(11) **EP 4 495 102 A1**
(43) Date of publication of application: **22.01.2025**
(21) Application number: 22932866.1
(22) Date of filing: 11.05.2022
(51) Int. Cl.: C07D 221/18, C07D 217/18, A61P 3/06, A61P 3/04

(54) **SYNTHESIS METHOD FOR NUCIFERINE OR DERIVATIVE THEREOF, NUCIFERINE DERIVATIVE AND APPLICATION THEREOF**

(30) Priority: 22.03.2022 CN 202210284445
(71) Applicant: Huaqiao Univesity, Quanzhou, Fujian 362021 (CN); Bayecao Health Industry Research Institute (Xiamen) Co., Ltd., Xiamen, Fujian 361000 (CN)
(72) Inventor: TANG, Xuchong, Quanzhou, Fujian 362021 (CN); ZHAO, Yingwei, Quanzhou, Fujian 362021 (CN); LI, Xuan, Xiamen, Fujian 361000 (CN); CAO, Liping, Xiamen, Fujian 361000 (CN)
(74) Representative: Kirkpatrick
(86) International application number: PCT/CN2022/092097
(87) International publication number: WO 2023/178809

(57) **Abstract**

A synthesis method for nuciferine or a derivative thereof, a nuciferine derivative and an application thereof. A compound having a structure shown in formula IV and R-X undergo a Schotten-Baumann reaction, and then nuciferine having a structure shown in formula VI or a derivative thereof is obtained by means of carbon-hydrogen bond activation. Further, the compound having the structure as shown in formula IV is obtained by an acyl chlorination reaction, a nucleophilic substitution reaction, a Bischler-Napieralski reaction, and a reduction reaction with 2-bromophenylacetic acid as a starting material. The nuciferine derivative can be used for preparing lipid-lowering drugs. The compound having the structure shown in formula IV and the nuciferine derivative having the structure shown in formula VI are shown as follows: formula IV and formula VI.

## Description

This application claims the priority of a Chinese patent application No. 202210284445.1, entitled "Method for synthesizing nuciferine or derivative thereof, derivative of nuciferine and use thereof, and filed with the China National Intellectual Property Administration on March 22, 2022, which is incorporated by reference herein in its entirety.

### TECHNICAL FIELD

The disclosure relates to the technical field of drug synthesis, in particular to a method for synthesizing nuciferine or a derivative thereof, as well as a derivative of nuciferine and use thereof.

### BACKGROUND

Obesity is a metabolic syndrome caused by the accumulation of fat due to excessive intake of energy and the destruction of the balance of energy metabolism. It is characterized by an abnormal increase in the percentage of body fat to body weight due to the increase in the volume and number of fat cells in the body and excessive deposition of fat in some local tissues. The pathogenesis of obesity is complex, including behavioral, environmental, physiological, genetic, social and economic factors. In recent years, the incidence of obesity has been increasing yearly throughout the world, and has become a worldwide health problem.

Lotus leaf is the leaf of a *Nymphaeaceae* Nelumbo plant, of which there are two species. One is *Nelumbo pentapetala Fernald,* distributed in North America with the northeastern of United States as the center; the other is *Nelumbo nucifera Gaertn,* distributed in Asia and Oceania with China as the center. There is only *Nelumbo nucifera Gaertn* in China. It is a perennial aquatic herb, which is widely cultivated in Hunan, Hubei, Zhejiang, Jiangsu provinces and other places.

According to Compendium of Materia Medica, "taking lotus leaves could help lose weight". Since ancient times, lotus leaves play a good role in reducing fat and losing weight. Traditional medicine believes that lotus leaves are bitter and mild, and attributed to liver, spleen and stomach channels; and have the functions of clearing heat and dampness, raising Yang, clearing away the heart fire, stopping bleeding and removing water. Lotus leaves are rich in nutrition, in addition to the common carbohydrates, lipids, proteins, and other conventional components of ordinary plants, but also rich in alkaloids and flavonoids with obvious biological activities. In recent years, the separation engineering and pharmacological activities of the chemical components of lotus leaves have been studied. Pure compounds of various alkaloid compounds have been isolated from the alkaloid components of lotus leaves, and it has been found that alkaloid compounds have the effects of lipid-lowering, weight losing, bacteriostatic, antiviral, and anticonvulsant.

Since the 1960s, several alkaloids have been gradually isolated from lotus leaves, mainly including nuciferine, O-nornuciferine, roemerine, N-norarmepavine, armepavine, anonaine, lirioderine, and N-methylcoclaurine. These alkaloids all have a structure similar to nuciferine, and all contain tetrahydroisoquinoline (THIQ) or isoquinoline structure.

So far, the acquisition of alkaloids from lotus leaves is mainly realized by solvent extraction, and studies on pharmacological efficacy of lotus leaves have been concentrated on the pharmaceutical effects of various crude extracts. The results showed that the total alkaloids of lotus leaf significantly inhibited the weight gain of obese rats, and decreased the serum total cholesterol (TC), triglyceride (TG) and atherosclerosis index (AI) of obese hyperlipidemia rats.

From the existing studies, the total alkaloids of lotus leaves have great lipid-regulating effects and are of great development value. However, the research on its lipid-lowering mechanism is still very limited, and the existing literature has only made a simple exploration. On the other hand, it is still a blank to recognize the hypolipidemic effect of nuciferine at the molecular level, and the studies on the structure-activity relationship of the hypolipidemic effect of nuciferine molecule have not been carried out. The main reason for the above situation is that acquisition of the alkaloids in lotus leaves are only dependent on plant extracts, and it is difficult to structurally modify the alkaloids, thus making it difficult to study their lipid-lowering effects from the point of molecular structure.

Organic synthesis enables the synthesis of a single component and is able to obtain the corresponding compound according to the designed structure, and thus providing powerful support for the scientific utilisation of natural products, the evaluation of the pharmacological activity of each component, and the development of new drug leading compounds. However, there are currently no effective chemical synthesis methods for nuciferine or a derivative thereof in the art.

### SUMMARY

An object of the present disclosure is to provide a method of synthesizing nuciferine or a derivative thereof, as well as a derivative of nuciferine and use thereof. The method according to the present disclosure is capable of synthesizing nuciferine and could flexibly achieve the structural modification to nuciferine, which is of great significance for establishing the structure-activity relationship of nuciferine drugs and thoroughly investigating the lipid-lowering mechanism of nuciferine.

In order to achieve the object above, the present disclosure provides the following technical solutions:

Provided is a method for synthesizing nuciferine or a derivative thereof, including the steps of
(1) mixing a compound having a structure shown in formula IV, R-X, and an alkaline catalyst, and subjecting a resulting mixture to Schotten-Baumann reaction, to obtain a compound having a structure shown in formula V, wherein in the R-X, R represents aliphatic hydrocarbyl, aromatic hydrocarbyl, or acyl, and X represents halogen; and 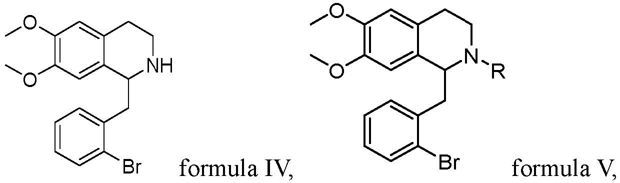
(2) mixing the compound having the structure shown in formula V, a phosphine ligand compound, an alkaline reagent, and a catalyst, and subjecting a resulting mixture to carbon-hydrogen bond activation reaction, to obtain nuciferine or the derivative thereof, wherein
   the nuciferine or the derivative thereof has a structure shown in formula VI, and 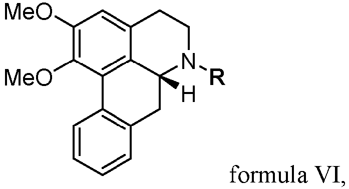
   under the condition that R represents methyl, the compound shown in formula VI is the nuciferine, and under the condition that R represents other group, the compound shown in formula VI is the derivative of nuciferine.

In some embodiments, the alkaline catalyst in step (1) includes at least one selected from the group consisting of an alkali metal hydroxide, an alkali metal carbonate, an alkali metal alkoxide, an amine compound, an ammonium salt, and an aminopyridine compound; and a molar ratio of the compound having the structure shown in formula IV to the alkaline catalyst ranges from 1 : 1 to 1 : 2.

In some embodiments, a molar ratio of the compound having the structure shown in formula IV to the R-X ranges from 1 : 1 to 1 : 3.

In some embodiments, the Schotten-Baumann reaction is performed at a temperature of 25-40 °C for 2-5 h.

In some embodiments, the alkaline reagent in step (2) includes at least one selected from the group consisting of an alkali metal carbonate, an alkali metal bicarbonate, and an alkali metal hydroxide; and a molar ratio of the compound having the structure shown in formula V to the alkaline reagent ranges from 1 : 2 to 1 : 4.

In some embodiments, the catalyst in step (2) includes at least one selected from the group consisting of nickel chloride, bis(triphenylphosphine)nickel chloride, palladium chloride, tetra(triphenylphosphine)palladium, palladium acetate, and palladium on carbon; and an amount in moles of the catalyst is 3-10% of an amount in moles of the compound having the structure shown in formula V

In some embodiments, an amount in moles of the phosphine ligand compound is 10-30% of an amount in moles of the compound having the structure shown in formula V

In some embodiments, an amount in moles of the phosphine ligand compound is 10-30% of an amount in moles of the compound having the structure shown in formula V

In some embodiments, the phosphine ligand compound includes at least one selected from the group consisting of triphenylphosphine, tributylphosphine, tri-t-butylphosphine, tri-(o-methylphenyl)phosphine, tri-(p-tolyl)phosphine, and tri-(m-tolyl)phosphine.

In some embodiments, the carbon-hydrogen bond activation reaction is performed at a temperature of 120-150 °C for 8-15 h.

In some embodiments, the compound having the structure shown in formula V is prepared by a process including the following steps:
(i) mixing 2-bromophenylacetic acid, an acyl chlorination reagent, and a catalyst, subjecting a resulting mixture to acyl chlorination reaction, to obtain a compound having a structure shown in formula I; 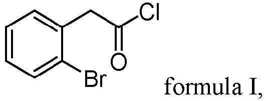
(ii) mixing the compound having the structure shown in formula I, 3,4-dimethoxyphenethylamine, and an alkaline reagent, and subjecting a resulting mixture to nucleophilic substitution reaction, to obtain a compound having a structure shown in formula II; 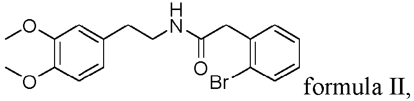
(iii) mixing the compound having the structure shown in formula II and POCl₃, and subjecting a resulting mixture to Bischler-Napieralski reaction, to obtain a compound having a structure shown in formula III; and 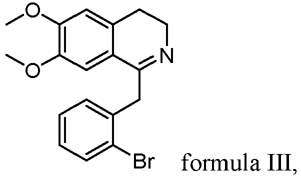
(iv) mixing the compound having the structure shown in formula III with a reducing agent, and subjecting a resulting mixture to reduction reaction, to obtain the compound having the structure shown in formula IV

In some embodiments, the acyl chlorination reagent in step (i) includes at least one selected from the group consisting of phosphorus trichloride, phosphorus pentachloride, sulfoxide chloride, oxalyl chloride, and triphosgene; and a molar ratio of 2-bromophenylacetic acid to the acyl chlorination reagent ranges from 1 : 1 to 1 : 3.

In some embodiments, the catalyst in step (i) includes at least one selected from the group consisting of N,N-dimethylformamide, ferric chloride, imidazole and aluminum chloride; and the catalyst is in an amount of 5% to 20% of a weight of 2-bromophenylacetic acid.

In some embodiments, the acyl chlorination reaction is performed at a temperature of 20-50 °C for 2-5 h.

In some embodiments, the alkaline reagent in step (ii) includes at least one selected from the group consisting of an alkali metal carbonate, an alkali metal hydroxide, triethylamine, and pyridine; and a molar ratio of the alkaline reagent to the 3,4-dimethoxyphenethylamine ranges from 1 : 1 to 3 : 1.

In some embodiments, a molar ratio of 3,4-dimethoxyphenethylamine to the compound having the structure shown in formula I ranges from 1 : 1 to 5 : 1.

In some embodiments, the nucleophilic substitution reaction is performed at a temperature of 25-40 °C for 8-15 h.

In some embodiments, in step (iii), a molar ratio of the compound having the structure shown in formula II to POCl₃ ranges from 1 : 3 to 1 : 8. In some embodiments, the Bischler-Napieralski reaction is performed at a temperature of 35-60 °C for 8-15 h.

In some embodiments, the reducing agent in step (iv) includes at least one selected from the group consisting of sodium borohydride, potassium borohydride, lithium aluminum hydride, and borane; and a molar ratio of the compound having the structure shown in formula III to the reducing agent ranges from 1 : 1 to 1 : 3.

In some embodiments, the reduction reaction is performed at a temperature of 20-40 °C for 2-5 h.

The disclosure also provides a derivative of nuciferine, having a structure shown in formula VI-1. 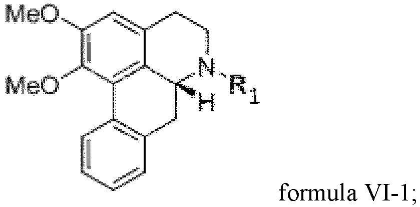 wherein in formula VI-1, Ri represents non-methyl aliphatic hydrocarbyl, aromatic hydrocarbyl, or acyl.

In some embodiments, R₁ represents formyl, benzoyl, or a substituted benzoyl.

In some embodiments, the substituted benzoyl is one selected from the group consisting of p-fluorobenzoyl, p-methylbenzoyl, o-methylbenzoyl, and p-methoxybenzoyl.

The disclosure also provides use of the derivative of nuciferine in preparation of a lipid-lowering drug.

In some embodiments, the lipid-lowering drug is an antihyperlipidemic drug.

The disclosure provides a method for treating hyperlipidemia, including

administrating the derivative of nuciferine as described in above technical solutions.

In some embodiments, the derivative of nuciferine is administrated at a dose of 20 mg/ (kg·d).

The present disclosure also provides a method for synthesizing nuciferine or a derivative thereof. In the synthesis method, a compound having a structure of formula IV and R-X are subjected to Schotten-Baumann reaction to obtain a compound having a structure of formula V The compound having a structure of formula V is subjected to C-H bond activation to obtain nuciferine or a derivative thereof. The synthesis method according to the present disclosure has simple steps, easy operations, and easily available raw materials. The most critical step in the synthesis route of nuciferine or a derivative thereof is to construct the carbon-carbon bond in the nuciferine molecule. The C-H bond activation reaction in the present disclosure is efficient, direct and flexible, and highly suitable for the synthesis of nuciferine molecule. In the present disclosure, the C-H functionalization reaction is successfully applied to the synthesis of natural products, which could not only greatly shorten the synthesis steps, but also rapidly expand to derivatives on the basis of the molecular structure of the original natural product, in order to screen out more active drug molecules.

Further, in the disclosure, 2-bromophenylacetic acid is used as the starting material to obtain a compound having a structure shown in formula I through acyl chlorination reaction, and the compound having a structure shown in formula I and 3,4-dimethoxyphenethylamine are subjected to nucleophilic substitution reaction, to obtain a compound having a structure shown in formula II. The compound having a structure shown in formula II is subjected to Bischler-Napieralski reaction and reduction reaction, to obtain the compound having a structure shown in formula IV The compound having a structure shown in formula IV according to the disclosure is synthesized with simple and efficient steps.

The method according to the present disclosure allows for convenient synthesis of nuciferine, and flexible structure modifications of nuciferine, so as to obtain a series of derivative of nuciferine. On this basis, the present disclosure comprehensively evaluates the lipid-lowering activity of nuciferine molecules through cell and animal experiments, and provides a solid foundation for developing nuciferine drugs to treat obesity and hyperlipidemia.

The present disclosure also provides a derivative of nuciferine. In present disclosure, network pharmacology and molecular docking technology are adopted to guide the rational structural modification of nuciferine, to obtain a series of derivatives of nuciferine with higher activity to obesity targets, which have broad application prospects in the preparation of lipid-lowering drugs. The docking of nuciferine molecules with related targets could provide reference for the treatment of obesity, which thereby deepens the understanding of the lipid-lowering mechanism of nuciferine.

### BRIEF DESCRIPTION OF THE DRAWINGS

FIG. 1 shows an integrated Venn diagram of disease targets.
FIG. 2 shows a Venn diagram of drug target-disease target.
FIG. 3 shows final results of topology screening of PPI network.
FIG. 4 shows GO enrichment results of common targets of active component(s) in a lotus leaf when treating obesity.
FIG. 5 shows KEGG pathway enrichment results of potential targets of main active components in a lotus leaf when treating obesity.
FIG. 6 shows an ¹H-NMR spectrum of compound 6a.
FIG. 7 shows a ¹³C-NMR spectrum of compound 6a.
FIG. 8 shows photographs of liver tissues of hyperlipidemia rats.
FIG. 9 shows results of HE stained liver tissues of hyperlipidemia rats.
FIG. 10 shows results of oil red O stained liver tissues of hyperlipidemia rats.
FIG. 11 shows the expression of IL-6 in liver tissues of hyperlipidemia rats.
FIG. 12 shows the expression of TNF-α in liver tissues of hyperlipidemia rats.

### DETAILED DESCRIPTION OF THE EMBODIMENTS

The disclosure provides a method for synthesizing nuciferine or a derivative thereof, including the following steps:
(1) mixing a compound having a structure shown in formula IV, R-X, and an alkaline catalyst, and subjecting a resulting mixture to Schotten-Baumann reaction, to obtain a compound having a structure shown in formula V, wherein in the R-X, R represents aliphatic hydrocarbyl, aromatic hydrocarbyl, or acyl, and X represents halogen; and 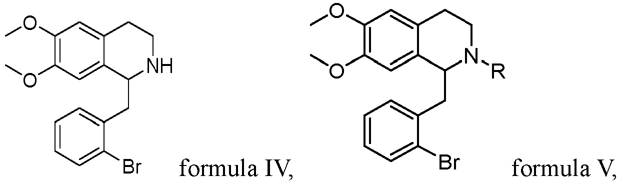
(2) mixing the compound having the structure shown in formula V, a phosphine ligand compound, an alkaline reagent, and a catalyst, and subjecting a resulting mixture to carbon-hydrogen bond activation reaction, to obtain the nuciferine or the derivative thereof, wherein
   the nuciferine or the derivative thereof has a structure shown in formula VI, and 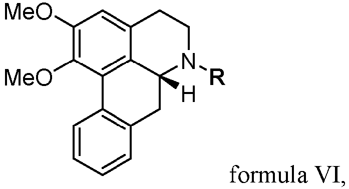
   under the condition that R represents methyl, the compound shown in formula VI is the nuciferine, and under the condition that R represents other group, the compound shown in formula VI is the derivative of nuciferine.

In the present disclosure, the compound having a structure shown in formula IV, R-X, and an alkaline catalyst are mixed, and a resulting mixture is subjected to Schotten-Baumann reaction, to obtain a compound having a structure shown in formula V In some embodiments of the present disclosure, the compound having a structure shown in formula IV is prepared by a process including the following steps:
(i) mixing 2-bromophenylacetic acid, an acyl chlorination reagent, and a catalyst, subjecting a resulting mixture to acyl chlorination reaction, to obtain a compound having a structure shown in formula I; 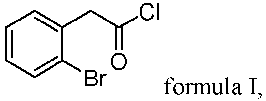
(ii) mixing the compound having the structure shown in formula I, 3,4-dimethoxyphenethylamine, and an alkaline reagent, and subjecting a resulting mixture to nucleophilic substitution reaction, to obtain a compound having a structure shown in formula II; 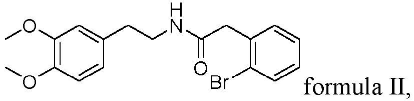
(iii) mixing the compound having the structure shown in formula II and POCl₃, and subjecting a resulting mixture to Bischler-Napieralski reaction, to obtain a compound having a structure shown in formula III; and 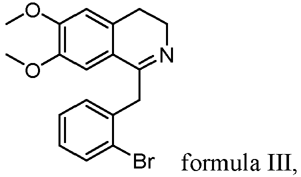
(iv) mixing the compound having the structure shown in formula III with a reducing agent, and subjecting a resulting mixture to reduction reaction, to obtain the compound having the structure shown in formula IV

In some embodiments of the disclosure, 2-bromophenylacetic acid, the acyl chlorination reagent, and the catalyst are mixed and a resulting mixture is subjected to acyl chlorination reaction, to obtain the compound having a structure shown in formula I. In some embodiments of the present disclosure, the acyl chlorination reagent includes at least one of phosphorus trichloride, phosphorus pentachloride, sulfoxide chloride, oxalyl chloride, and triphosgene. In some embodiments, a molar ratio of 2-bromophenylacetic acid to the acyl chlorination reagent ranges from 1 : 1 to 1 : 3, and preferably 1 : (1.5-2.5). In some embodiments, the catalyst includes at least one of N,N-dimethylformamide, ferric chloride, imidazole, and aluminum chloride. In some embodiments, the catalyst is in an amount of 5 to 20% of a weight of 2-bromophenylacetic acid, and preferably 10-15%.

In some embodiments of the present disclosure, the acyl chlorination reaction is carried out in an organic solvent, and the organic solvent preferably includes at least one of dichloromethane, chloroform, methanol, ethanol, acetonitrile, and tetrahydrofuran. In some embodiments, a ratio of a mass of 2-bromophenylacetic acid to a volume of the organic solvent is in a range of 1 g : (5-10) mL.

In some embodiments of the present disclosure, the acyl chlorination reaction is performed at a temperature of 20-50 °C and preferably 30-40 °C. In some embodiments, the acyl chlorination reaction is performed for 2-5 h, and preferably 3-4 h.

In specific embodiments of the present disclosure, 2-bromophenylacetic acid is dissolved in an organic solvent; an acyl chlorination reagent and a catalyst are then added to the resulting solution; and the resulting mixture is subjected to reaction at an acyl chlorination reaction temperature. In some embodiments, the acyl chlorination reaction is carried out under a stirring condition. In the present disclosure, there is no special requirement for specific operations of stirring.

In some embodiments of the present disclosure, after the acyl chlorination reaction, the reaction liquid obtained from the acyl chlorination reaction is subjected to post-treatment, to obtain a compound having a structure shown in formula I. In some embodiments of the present disclosure, the post-treatment includes the following steps: subjecting the reaction liquid obtained from the acyl chlorination reaction to rotary evaporation to remove the organic solvent therein, to obtain the compound having a structure shown in formula I, wherein preferably the rotary evaporation is performed at a temperature of 35-45 °C. In the present disclosure, there is no special requirements for the rotary evaporation time, as long as the solvent could be removed.

In some embodiments, after obtaining the compound having a structure shown in formula I, the compound having a structure shown in formula I, 3,4-dimethoxyphenethylamine, and an alkaline reagent are mixed and a resulting mixture was subjected to nucleophilic substitution reaction, to obtain the compound having a structure shown in formula II. In some embodiments of the present disclosure, the alkaline reagent includes at least one of an alkali metal carbonate, an alkali metal hydroxide, triethylamine, and pyridine; preferably the alkali metal carbonate is sodium carbonate or potassium carbonate; preferably, the alkali metal hydroxide is sodium hydroxide or potassium hydroxide. In some embodiments, a molar ratio of the alkaline reagent to 3,4-dimethoxyphenethylamine is in a range of (1-3) : 1, and preferably (1.5-2.5) : 1. In some embodiments, a molar ratio of 3,4-dimethoxyphenethylamine to the compound having a structure shown in formula I is in a range of (1-5) : 1, and preferably (2-4) : 1. In some embodiments, the nucleophilic substitution reaction is carried out in an organic solvent, and preferably the organic solvent is of the same type as that used in the acyl chlorination reaction, which will not be repeated herein. In some embodiments, a volume ratio of 3,4-dimethoxyphenethylamine to the organic solvent is in a range of 1 : (5-15).

In some embodiments of the present disclosure, the nucleophilic substitution reaction is performed at a temperature of 25-40 °C, and preferably 30-35 °C. In some embodiments, the nucleophilic substitution reaction is performed for 8-15 h, and preferably 9-14 h.

In some specific embodiments of the present disclosure, 3,4-dimethoxyphenethylamine and an alkaline reagent are dissolved in an organic solvent, and at 0-5 °C, the compound having a structure shown in formula I is added thereto; after the addition, a resulting mixture is heated to a temperature for the nucleophilic substitution reaction and subjected to the nucleophilic substitution reaction. In some embodiments, the nucleophilic substitution reaction is performed under stirring. In the present disclosure, there is no special requirement for specific operations of stirring.

In some embodiments of the present disclosure, after the nucleophilic substitution reaction, the reaction liquid obtained from the nucleophilic substitution reaction is subjected to post-treatment, to obtain the compound having a structure shown in formula II. In some embodiments of the present disclosure, the post-treatment includes the following steps: quenching the nucleophilic substitution reaction, then subjecting the resulting reaction liquid to extraction, to obtain an extraction phase, dewatering and filtering the extraction phase in sequence, and removing the extractant, to obtain the compound having a structure shown in formula II. In some embodiments of the disclosure, an agent used for quenching is brine. In some embodiments, a volume ratio of the brine to the reaction liquid ranges from 1 : 1 to 2 : 1. There is no special requirement on the concentration of the brine, as long as the reaction could be quenched In some embodiments of the present disclosure, the agent for extraction is dichloromethane. In some embodiments, a volume ratio of dichloromethane to the reaction liquid ranges from 2 : 1 to 3 : 1. In some embodiments of the present disclosure, the reaction liquid after quenching is subjected to extraction, and the compound having a structure shown in formula II thereby enters the extraction phase. In some embodiments of the present disclosure, a solid dehydrating agent is used to dehydrate the extraction phase. In some embodiments, the solid dehydrating agent is at least one of anhydrous sodium sulfate, solid sodium hydroxide, solid calcium oxide, anhydrous calcium chloride, anhydrous potassium carbonate, and anhydrous magnesium sulfate. In some embodiments, a ratio of a mass of the solid dehydrating agent to a volume of the extraction phase is in a range of 1 g : (10-15) mL. In some embodiments, after dehydration is completed, the solid dehydrating agent is removed by filtration. In the present disclosure, there is no special requirement for the filtration. In some embodiments, the extractant is removed by rotary evaporation, to obtain the solid compound having a structure shown in formula II. In some embodiments of the disclosure, the rotary evaporation is performed at a temperature of 35-45 °C. In the present disclosure, there is no special requirements on the time of the rotary evaporation, as long as the extractant could be fully removed.

In some embodiments, after the compound having a structure shown in formula II is obtained, the compound having a structure shown in formula II is mixed with POCl₃, and a resulting mixture is subjected to Bischler-Napieralski reaction, to obtain the compound having a structure shown in formula III. In some embodiments of the present disclosure, a molar ratio of the compound having a structure shown in formula II to POCl₃ is in a range of 1 : (3-8), and preferably 1 : (4-7). In some embodiments, the Bischler-Napieralski reaction is carried out in an organic solvent, and preferably the organic solvent is of the same type as that used in the acyl chlorination reaction, which will not be repeated herein. In some embodiments, a volume ratio of the compound having a structure shown in formula II to the organic solvent is in a range of 1 g : (5-15) mL.

In some embodiments of the present disclosure, the Bischler-Napieralski reaction is performed at a temperature of 35-60 °C, and preferably 40-55 °C. In some embodiments, the Bischler-Napieralski reaction is performed for 8-15 h, and preferably 9-12 h.

In some specific embodiments of the disclosure, the compound having a structure shown in formula II is dissolved in an organic solvent, POCl₃ is then added thereto, followed by stirring, and a resulting mixture is subjected to reaction. In some embodiments, the Bischler-Napieralski reaction is carried out under stirring. In the persent disclosure, there is no special requirement for specific operations of stirring.

In some embodiments, after the Bischler-Napieralski reaction is completed, the reaction liquid obtained from the Bischler-Napieralski reaction is subjected to post-treatment, to obtain the compound having a structure shown in formula III. In some embodiments of the present disclosure, the post-treatment includes the following steps: cooling the reaction liquid obtained from the Bischler-Napieralski reaction to 0 °C, quenching, subjecting the reaction liquid after quenching to extraction, and performing extraction phase dehydration, filtration, and removal of extractant in sequence. In some embodiments of the present disclosure, the reaction system is cooled to 0 °C to prevent excessive reaction during subsequent quenching. In some embodiments of the present disclosure, the agent used for quenching is water. In some embodiments, a volume ratio of water to the reaction liquid ranges from 1 : 1 to 2 : 1. In some embodiments, the reaction liquid after quenching is subjected to extraction, and the compound having a structure shown in formula III thereby enters the extraction phase. In some embodiments of the present disclosure, the extractant is dichloromethane, and preferably a volume ratio of dichloromethane to the reaction liquid ranges from 2 : 1 to 3 : 1. In the present disclosure, the extraction phase is dehydrated with a solid dehydrating agent. In some embodiments, the solid dehydrating agent is at least one of anhydrous sodium sulfate, solid sodium hydroxide, solid calcium oxide, anhydrous calcium chloride, anhydrous potassium carbonate, and anhydrous magnesium sulfate. In some embodiments, a ratio of a mass of the solid dehydrating agent to a volume of the extraction phase is in a range of 1 g : (10-15) mL. In some embodiments, after dehydration, the solid dehydrating agent is removed by filtration, and there is no special requirement for the filtration. In some embodiments of the present disclosure, the extractant is removed by rotary evaporation to obtain the solid compound having a structure shown in formula III. In some embodiments of the present disclosure, the rotary evaporation is performed at a temperature of 35-45 °C. In the present disclosure, there is no special requirement for the time of the rotary evaporation.

In some embodiments, after the compound having a structure shown in formula III is obtained, the compound having a structure shown in formula III is mixed with the reducing agent, and a resulting mixture is subjected to reduction reaction, to obtain the compound having a structure shown in formula IV In some embodiments of the present disclosure, the reducing agent includes at least one of sodium borohydride, potassium borohydride, lithium aluminum hydride, and borane. In some embodiments, a molar ratio of the compound having a structure shown in formula III to the reducing agent is in a range of 1 : (1-3), and preferably 1 : (1.5-2.5). In some embodiments, the reduction reaction is carried out in an organic solvent, and preferably the organic solvent is of the same type as that used in the acyl chlorination reaction, which will not be repeated herein. In some embodiments, a volume ratio of the compound having a structure shown in formula III to the organic solvent is in a range of 1 g : (5-10) mL.

In some embodiments of the present disclosure, the reduction reaction is performed at a temperature of 20-40 °C, and preferably 25-35 °C. In some embodiments, the reduction reaction is performed for 2 to 5 h, and preferably 3 to 4 h.

In specific embodiments of the present disclosure, the compound having a structure shown in formula III is dissolved in an organic solvent, and a reducing agent is then added thereto, and a resulting mixture is subjected to reaction. In some embodiments, the reduction reaction is carried out under stirring, and there is no special requirement for specific operations of stirring.

In some embodiments of the present disclosure, after the reduction reaction is completed, the reaction liquid obtained from the reduction reaction is subjected to post-treatment, to obtain the compound having a structure shown in formula IV In some embodiments of the present disclosure, the post-treatment includes the following steps: quenching the reaction liquid obtained from the reduction reaction, performing concentration, extraction, dehydration of extraction phase, filtration, and removal of the extractant in sequence, to obtain the compound having a structure shown in formula IV In some embodiments of the present disclosure, the agent used for quenching is water. In some embodiments, a volume ratio of water to the reaction liquid range from 0.5 :1 to 1 : 1. In some embodiments of the disclosure, the reaction liquid is concentrated by rotary evaporation. In some embodiments, a volume ratio of the concentrated reaction liquid to the original reaction liquid is in a range of (0.2-0.5) : 1, and most of the solvent is removed by concentrating. In some embodiments of the present disclosure, the extractant is dichloromethane. In some embodiments, a volume ratio of dichloromethane to the reaction liquid is in a range of (2-3) : 1. In some embodiments, the concentrated reaction liquid is subjected to extraction, and the compound having a structure shown in formula IV thereby enters the extraction phase. In some embodiments of the present disclosure, the extraction phase is dehydrated with a solid dehydrating agent. In some embodiments, the solid dehydrating agent is at least one of anhydrous sodium sulfate, solid sodium hydroxide, solid calcium oxide, anhydrous calcium chloride, anhydrous potassium carbonate, and anhydrous magnesium sulfate. In some embodiments, a ratio of a mass of the solid dehydrating agent to a volume of the extraction phase is in a range of 1 g : (10-15) mL. In some embodiments, after dehydration, the solid dehydrating agent is removed by filtration, and there is no special requirements for the filtration. In some embodiments, the extractant is removed by rotary evaporation, to obtain a solid of the compound having a structure shown in formula IV In some embodiments, the rotary evaporation is performed at a temperature of 35-45 °C. In the present disclosure, there is no special requirements on the time of the rotary evaporation, as long as the extractant could be fully removed.

After obtaining the compound having a structure shown in formula IV, the compound having a structure shown in formula IV, R-X, and an alkaline catalyst are mixed, and a resulting mixture is subjected to Schotten-Baumann reaction, to obtain the compound having a structure shown in formula V In the disclosure, R in the R-X represents aliphatic hydrocarbyl, aromatic hydrocarbyl, or acyl. In some embodiments, the aliphatic hydrocarbyl is ethyl, propyl, cyclopropyl, or benzyl. In some embodiments, the aromatic hydrocarbyl is phenyl or naphthyl. In some embodiments, the acyl is acetyl, benzoyl, or substituted benzoyl. In some embodiments, the substituted benzoyl is p-fluorobenzoyl, p-methylbenzoyl, o-methylbenzoyl, or p-methoxybenzoyl. X in R-X represents halogen, preferably -F, -Cl, -Br, or -I. In some specific embodiments of the disclosure, the R-X is acetyl chloride, benzoyl chloride, p-fluorobenzoyl chloride, p-methylbenzoyl chloride, o-methylbenzoyl chloride, or p-methoxybenzoyl chloride. In addition, under the condition that the R-X is CH₃I, the finally synthesized product is nuciferine. In some embodiments, a molar ratio of the compound having a structure shown in formula IV to R-X is in a range of 1 : (1-3), and preferably 1 : (1.5-2.5).

In some embodiments of the present disclosure, the alkaline catalyst includes at least one of an alkali metal hydroxide, an alkali metal carbonate, an alkali metal alkoxide, an amine compound, an ammonium salt, and an aminopyridine compound. In some embodiments, the alkali metal hydroxide includes potassium hydroxide and/or sodium hydroxide. In some embodiments, the alkali metal carbonate includes potassium carbonate and/or sodium carbonate. In some embodiments, the alkali metal alkoxide includes sodium methoxide and/or potassium tert-butoxide. In some embodiments, the amine compound is triethylamine. In some embodiments, the ammonium salt is ammonium acetate. In some embodiments, the aminopyridine compound is 4-dimethylaminopyridine (DMAP). In some embodiments, a molar ratio of the compound having a structure shown in formula IV to the alkaline catalyst is in a range of 1 : (1-2), and preferably 1 : (1.3-1.5).

In some embodiments of the present disclosure, the Schotten-Baumann reaction is carried out in an organic solvent. In some embodiments, the organic solvent is of the same type as that used in the acyl chlorination reaction, which will not be repeated herein. In some embodiments, a ratio of the compound having a structure shown in formula IV to a volume of the organic solvent is in a range of 1 g : (3-6) mL.

In some embodiments of the present disclosure, the Schotten-Baumann reaction is performed at a temperature of 25-40 °C, and preferably 30-35 °C. In some embodiments, the Schotten-Baumann reaction is performed for 2-5 h, and preferably 3-4 h.

In some specific embodiments of the disclosure, the compound having a structure shown in formula IV and the alkaline catalyst are dissolved in an organic solvent, compound R-X is then added thereto, and a resulting mixture is subjected to reaction. In some embodiments, the Schotten-Baumann reaction is carried out under stirring, and in the present disclosure there is no special requirement for specific operations of stirring.

In some embodiments, after the Schotten-Baumann reaction is completed, the reaction liquid obtained from the Schotten-Baumann reaction is subjected to post-treatment, to obtain the compound having a structure shown in formula V In some embodiments of the present disclosure, the post-treatment includes the following steps: quenching the reaction liquid obtained from the Schotten-Baumann reaction, and performing extraction, dehydration of extraction phase, filtration, and removal of extractant in sequence. In some embodiments of the present disclosure, an agent used for quenching reaction is water. In some embodiments, a volume ratio of water to the reaction liquid is in a range of (1-2) : 1. In some embodiments of the present disclosure, the extractant is dichloromethane. In some embodiments, a volume ratio of dichloromethane to the reaction liquid is in a range of (2-3) : 1. In some embodiments, the compound having a structure shown in formula V enters the extraction phase through extraction. In some embodiments of the present disclosure, a solid dehydrating agent is used to dehydrate the extraction phase. In some embodiments, the solid dehydrating agent is at least one of anhydrous sodium sulfate, solid sodium hydroxide, solid calcium oxide, anhydrous calcium chloride, anhydrous potassium carbonate, and anhydrous magnesium sulfate. In some embodiments, a ratio of a mass of the solid dehydrating agent to a volume of the extraction phase is in a range of 1 g : (10-15) mL. In some embodiments, after dehydration, the solid dehydrating agent is removed by filtration, and in the present disclosure there is no special requirement for the filtration. In some embodiments, the extractant is removed by rotary evaporation. In some embodiments of the disclosure, the rotary evaporation is performed at a temperature of 35-45 °C. In the present disclosure, there is no special requirement on the time of the rotary evaporation, as long as the extractant could be fully removed.

After the compound having a structure shown in formula V is obtained, the compound having a structure shown in formula V, a phosphine ligand compound, an alkaline reagent, and a catalyst are mixed, and a resulting mixture is subjected to carbon-hydrogen bond activation reaction, to obtain nuciferine or a derivative thereof. In the present disclosure, the nuciferine or the derivative thereof has a structure shown in formula VI, under the condition that R represents methyl, the compound shown in formula VI is nuciferine; and under the condition that R represents other group, the compound shown in formula VI is the derivative of nuciferine. In some embodiments of the present disclosure, the alkaline reagent includes at least one of an alkali metal carbonate, an alkali metal bicarbonate, and an alkali metal hydroxide. In some embodiments, the alkali metal carbonate includes potassium carbonate and/or sodium carbonate. In some embodiments, the alkali metal bicarbonate includes potassium bicarbonate and/or sodium bicarbonate. In some embodiments, the alkali metal hydroxide includes potassium hydroxide and/or sodium hydroxide. In some embodiments, a molar ratio of the compound having a structure shown in formula V to the alkaline reagent is in a range of 1 : (2-4), and preferably 1 : (2.5-3.5).

In some embodiments of the present disclosure, the catalyst includes at least one of nickel chloride, bis(triphenylphosphine)nickel chloride, palladium chloride, tetra(triphenylphosphine)palladium, palladium acetate, and palladium on carbon. In some embodiments, the catalyst is in an amount of 3 to 10%, preferably 5 to 8% of an amount in moles of the compound having a structure shown in formula V

In some embodiments of the present disclosure, the phosphine ligand compound includes at least one of triphenylphosphine (PPh₃), tributylphosphine, tri-t-butylphosphine, tri-(o-methylphenyl)phosphine, tri-(p-tolyl)phosphine, and tri-(m-tolyl)phosphine, and preferably is any one of triphenylphosphine, tributylphosphine, and tri-t-butylphosphine. In some embodiments, the phosphine ligand compound is in an amount of 10 to 30%, preferably 15 to 25% of an amount in moles of the compound having a structure shown in formula V

In some embodiments of the present disclosure, the carbon-hydrogen bond activation reaction is carried out in an organic solvent. In some embodiments, the organic solvent includes at least one of dichloromethane, tetrahydrofuran, N,N-dimethylacetamide, N,N-dimethylformamide, methylpyrrolidone, and dimethyl sulfoxide. In some embodiments, a dosage ratio of the compound having a structure shown in formula V to the organic solvent is in a range of 1 g : (5-10) mL.

In some specific embodiments of the disclosure, the compound having a structure shown in formula V, a phosphine ligand compound, an alkaline reagent, and a catalyst are dissolved in an organic solvent, and a resulting mixture is subjected to carbon-hydrogen bond activation reaction. In the present disclosure, there is no requirement for the dissolution order of the compound having a structure shown in formula V, the phosphine ligand compound, the alkaline reagent, and the catalyst. In some embodiments of the disclosure, the carbon-hydrogen bond activation reaction is carried out under stirring, and in the present disclosure there is no special requirement for specific operations of stirring.

In some embodiments, after the carbon-hydrogen bond activation reaction is completed, the reaction liquid obtained from the carbon-hydrogen bond activation reaction is subjected to post-treatment, to obtain nuciferine or the derivative thereof. In some embodiments of the present disclosure, the post-treatment includes the following steps: subjecting the reaction liquid obtained from the carbon-hydrogen bond activation reaction to extraction, performing dehydration of extraction phase, filtration, and removal of extractant in sequence. In some embodiments of the present disclosure, the extractant is dichloromethane. In some embodiments, a volume ratio of dichloromethane to the reaction liquid is in a range of (1-3) : 1. In some embodiments of the present disclosure, the extraction phase is dehydrated with a solid dehydrating agent. In some embodiments, the solid dehydrating agent is at least one of anhydrous sodium sulfate, solid sodium hydroxide, solid calcium oxide, anhydrous calcium chloride, anhydrous potassium carbonate, and anhydrous magnesium sulfate. In some embodiments, a ratio of a mass of the solid dehydrating agent to a volume of the extraction phase is in a range of 1 g : (10-15) mL. In some embodiments, after dehydration, the solid dehydrating agent is removed by filtration, and in the present disclosure there is no special requirement for the filtration. In some embodiments, the extractant is removed by rotary evaporation. In some embodiments of the disclosure, the rotary evaporation is performed at a temperature of 35-45 °C. In the present disclosure, there is no special requirement on the time of the rotary evaporation, as long as the extractant could be fully removed.

The present disclosure also provides a derivative of nuciferine, having a structure shown in formula VI-1: 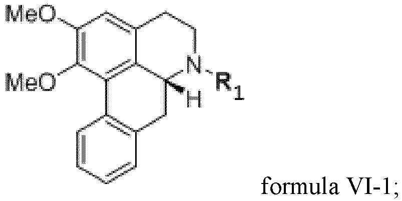 wherein in formula VI-1, Ri represents non-methyl aliphatic hydrocarbyl, aromatic hydrocarbyl, or acyl.

In some embodiments of the disclosure, R₁ in formula V-1 is not methyl, and the specific optional other type is consistent with that of R in formula VI, which will not be repeated herein. In particular embodiments, Ri in formula VI-1 represents formyl, benzoyl, or substituted benzoyl. In some embodiments, the substituted benzoyl is p-fluorobenzoyl, p-methylbenzoyl, o-methylbenzoyl, or p-methoxybenzoyl.

In the disclosure, the derivative of nuciferine is screened out by network pharmacology and molecular docking technology. Network pharmacology is a method to integrate multiple drug information based on network database analysis, find drug targets and compounds, understand multiple mechanisms of drug, and reduce the cost of drug preliminary screening. Molecular docking technology is a process that combines structural molecular biology and computer-aided drug design to find the best matching mode by complementing the spatial structure of the receptor active site and minimizing the binding energy. On the basis of the establishment of network pharmacology, the structure of nuciferine is modified, several possible structures are designed according to experience, and the parameters are set by using Autodock vina 1.5.6 for molecular docking. The molecules exhibiting good binding force with most of the relevant target proteins in the docking results are analyzed, which may be the active components with the highest predicted target Degree among the several modified structures of nuciferine. On this basis, the design scheme is further adjusted to obtain the derivatives of nuciferine according to in the disclosure.

In specific embodiments of the disclosure, the screening method includes the following steps:
(1) with oral bioavailability (OB)≥30% and drug-likeness (DL)≥0.18 as thresholds, searching in TCMSP database to obtain active components of lotus leaf; according to the obtained active components of lotus leaf, collecting gene names of targets of active components of lotus leaf using UniProt database, and deleting ineffective and repeated targets, to obtain targets of effective active components of lotus leaf.
(2) screening out obesity-related genes from Gene Card database, OMIM database, Mala Cards database, Therapeutic Targets database, and Drug Bank database, deleting duplicated targets, and taking the intersection of these databases, to obtain all the gene targets of obesity, as the search results of obesity-related targets;
(3) matching predicted results of targets of effective components of lotus leaf obtained in step (1) with search results of obesity-related targets obtained in step (2), and selecting overlapping targets as common gene targets of lotus leaf and obesity;
(4) importing active components of lotus leaf and the selected common gene targets of lotus leaf and obesity into Cytoscape, and constructing a network of the active components of lotus leaf and obesity targets;
(5) importing the common gene targets of lotus leaf and obesity selected in step (3) into STRING database, constructing a protein interaction network, and screening out core targets with degree centrality (DC), betweenness centrality (BC), closeness centrality (CC), eigenvector centrality (EC), network centrality (NC), and local average connectivity (LAC) as indicators;
(6) subjecting the common gene targets of lotus leaf and obesity selected in step (3) to GO enrichment and KEGG enrichment, screening out the top 20 GO analysis results and the top 20 KEGG analysis results, repeatedly screening and manually selecting from the results according to correlation, and deleting duplicated targets from the selected targets and the core targets screened in step (5), to obtain core targets of obesity, as predictive targets to guide molecular docking;
(7) performing structural modification based on nuciferine, the effective component of lotus leaf, performing molecular docking using Autodock vina 1.5.6, and screening out the active components with the highest predicted target Degree.

In the disclosure, the molecular docking results show that benzoylnuciferine has good binding force and high activity with most of the related target proteins, and a series of derivative of nuciferines are synthesized based on the above results.

The disclosure also provides the application of the derivative of nuciferine in the scheme in the preparation of lipid-lowering drugs. There is no special requirements for the application, and it can be used to prepare the derivative of nuciferine into various dosage forms of lipid-lowering drugs by a method well known to those skilled technicians.

The schemes provided by the disclosure are described in detail in combination with embodiments below, but they cannot be understood as limiting the scope of protection of the disclosure.

### Example 1

### Network pharmacology and molecular docking technology

### 1. Materials and methods

Screening of drug composition and targets thereof: Pharmacokinetics, pharmacodynamics, omics, etc. were analyzed systematically by TCMSP(Traditional Chinese Medica System Pharmacology Database and Analysis Platform) software, and a complete system model was built to provide a platform for pharmacodynamic prediction, TCM pharmacology, and validation research. Further, it also provided new research methods for target discovery and new drug development. In the disclosure, the chemical composition of lotus leaves was searched according to the collection of TCMSP database, a unique systematic pharmacology platform of traditional Chinese medicine. With oral bioavailability (OB)≥30% and drug-likeness (DL)≥0.18 as thresholds, the corresponding compounds and corresponding targets thereof were screened out. The main steps were performed as follows.

Acquisition of targets with disease-related effects: obesity-related genes were searched and screened out in the following databases: Gene Card database, OMIM database, Mala Cards database, Therapeutic Targets database, and Drug Bank database. Duplicates were deleted from the obesity-related genes obtained from the above five databases, and all the gene targets of obesity were obtained by taking the intersection of the databases. A total of 9182 obesity gene targets were identified. The target prediction results of effective components of lotus leaf were matched with the search results of obesity-related targets, and overlapping targets were selected as the relevant targets of lotus leaf in the treatment of obesity. The Venne2.1 online tool was used to map the active component targets and disease targets of lotus leaf and to draw a Venn diagram. A total of 171 cross targets of lotus leaf and obesity were collected as common genes of lotus leaf in obesity treatment, which may be potential targets of lotus leaf in obesity treatment.

Construction of drug active components and disease target network: the active components of lotus leaf and selected obesity targets were input into Cytoscape to construct the active components and obesity target network of lotus leaf.

Protein interaction network construction and core gene screening: PPI (protein-protein interaction) refers to the process by which two or more protein molecules form a protein complex through non-covalent bonds. PPI and acquired networks are very important in most biological functions and processes. The STRING collects a large number of protein interactions and contains data confidence levels in a certain range (low confidence: <0.4; medium confidence: 0.4-0.7; high confidence: >0.7). On this basis, the common gene targets of lotus leaf and obesity were imported into STRING database. Using species-qualified Homo sapiens, the minimum protein interaction score was set to 0.900, and the non-interacting protein nodes in the network were hidden; the protein interaction network analysis was performed, and the updated results of TSV format were downloaded. This file has been imported into Cytoscape for topology attribute analysis. The topological properties of the nodes in the interactive network were then evaluated by using App CytoNCA to compute six parameters: degree centrality (DC), betweenness centrality (BC), closeness centrality (CC), eigenvector centrality (EC), network centrality (NC), and local average connectivity (LAC). These six parameters measure the importance of the nodes in the network and indicate the nature of the nodes in the network. A node with high DC, BC, CC, EC, NC, and LAC values means that it plays a very important role in the network. According to the topological property analysis results of the above PPI, targets equal to or larger than the median were selected as the core targets for screening twice. Based on the above findings, the core index of the network was obtained.

GO and KEGG enrichment analysis: first, the symbol of the core target was transformed into the Entrez gene ID. Various bioinformatic analyses and results visualization could be achieved using software R project. GO enrichment was performed based on core gene files (molecular function (MF), biological process (BP), cell component (CC)). In the programming language, "pvalueCutoff=0.05" and "qvalueCutoff=0.05" were set. The top 20 items with the highest enrichment were selected from the analysis results, which were displayed in the form of bar charts. Finally, the top 20 path maps of enrichment ranking were downloaded from the KEGG database by using rgui and path view. Repeated screening and manual selection were conducted on the results according to relevance.

Molecular docking verification of active components and targets: By constructing the target network of effective components for drugs and diseases, the drug-like components of lotus leaf and the targets of obesity were obtained. The 3D structure file of the selected nuciferine could be directly downloaded from PubChem database in SDF format, and the configuration with the lowest molecular free energy could be calculated by Chem3D software. Protein crystal structures of obesity-related targets were downloaded from the RCSB PDB database. The downloaded protein crystal structures were imported into PyMOL, and commands were input to delete water molecules and small molecular ligands in the protein. The corresponding protein chain in the crystal structure was selected, the center and scope of the box were defined by clicking "plugin", "Legacy Plugins", "Get Box Plugin", and the selected protein region was wrapped therein. Molecular docking was performed using Autodock vina 1.5.6.

### 2. Results and discussion

### 2.1 Screening of effective components in lotus leaves

Searching was conducted in the TCMSP database, with screening conditions: OB (> 30%) and DL (> 0.18). A total of 93 active components were found in lotus leaves, and 15 active components were finally screened out. Table 1 shows the screening results of effective components in lotus leaves, which mainly contains active components such as flavonoids, alkaloids, polysaccharides, volatile oils, and organic acids.

**Table 1 Screening results of effective components of lotus leaves**

| Mol ID | Name | OB/% | DL |
|---|---|---|---|
| MOL000098 | quercetin | 46.43 | 0.28 |
| MOL000354 | isorhamnetin | 49.6 | 0.31 |
| MOL000359 | Sitosterol | 36.91 | 0.75 |
| MOL000422 | kaempferol | 41.88 | 0.24 |
| MOL006405 | (1S)-1-(4-hydroxybenzyl)-2-methyl-3,4-dihydro-1H-isoquinoline-6,7-diol | 67.14 | 0.23 |
| MOL003578 | Cycloartenol | 38.69 | 0.78 |
| MOL007206 | Armepavine | 69.31 | 0.29 |
| MOL007207 | Machiline | 79.64 | 0.24 |
| MOL007210 | o-Nornuciferine | 33.52 | 0.36 |
| MOL007213 | Nuciferin | 34.43 | 0.4 |
| MOL007214 | (+)-Leucocyanidin | 37.61 | 0.27 |
| MOL007217 | leucodelphinidin | 30.02 | 0.31 |
| MOL007218 | Remerin | 40.75 | 0.52 |
| MOL000073 | ent-Epicatechin | 48.96 | 0.24 |
| MOL000096 | (-)-catechin | 49.68 | 0.24 |

### 2.2 Construction of effective active component database and disease indicator set

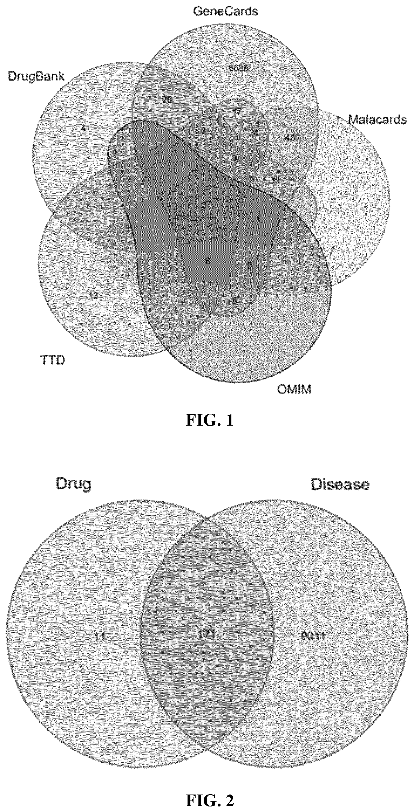

1193 active components of lotus leaf were obtained from TCMSP database. Gene names of targets were collected by using UniProt database, ineffective and duplicate targets therein were deleted, to obtain 182 effective active component targets. By searching in Gene Card database, OMIM database, Mala Cards database, Therapeutic Targets database and Drug Bank database and integrating, a total of 8953 obesity-related gene targets were obtained, as shown in FIG. 1. The intersection of 182 targets of active components of lotus leaf with 8953 obesity target genes were taken, obtaining 171 common target genes, as shown in FIG. 2.

### 2.3 Construction of the component-target network

The 15 selected compounds and 171 disease-related targets were constructed into a table, and the data were imported into Cytoscape to construct the component-target network. The final network had 185 nodes and 327 edges, with different nodes representing the active components of lotus leaf and the target of obesity. The composition-target network shows that lotus leaf could correspond to at least one active components of one target, and multiple targets could correspond to the same active component, indicating that lotus leaf has multi-component and multi-target characteristics in the treatment of obesity.

### 2.4 Target protein interaction network analysis

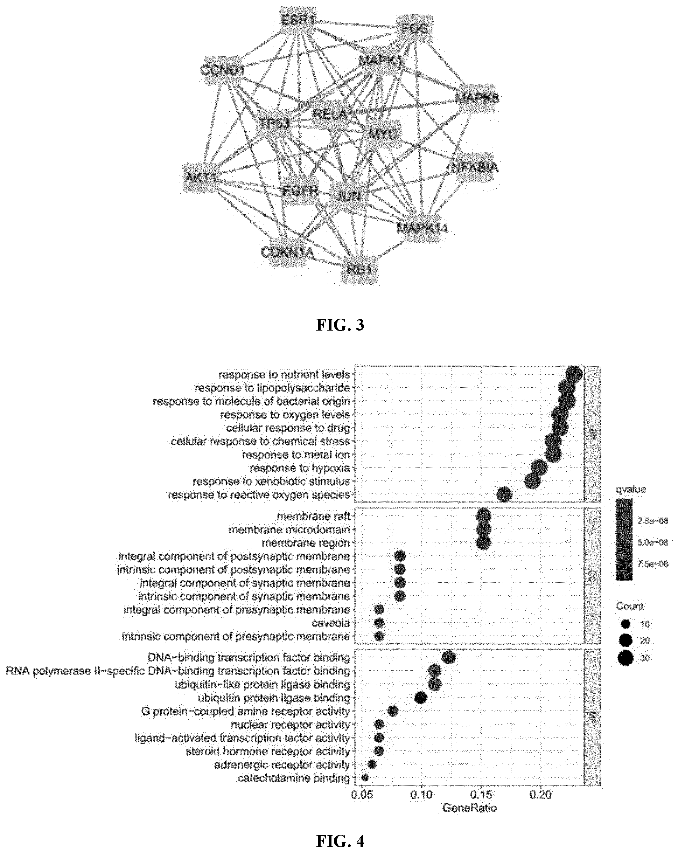

The common targets of lotus leaf and obesity treatment-related targets were imported into the STRING database to obtain the interaction relationship between them. The resulting interaction network had 153 nodes and 625 edges. The TSV data were imported into Cytoscape. CytoNCA analyzes and evaluates the topological properties of the nodes in the interactive network, with the DC, BC, CC, EC, NC, and LAC indices as screening indicators. The threshold values of the first screening were set as: DC>6, BC>78.21593442, LAC>2.4, CC>0.112927192, EC>0.043043971, NC>3.5. The results showed 46 nodes and 249 edges, and a total of 46 core genes were screened out. The threshold values of the second screening were set as: DC>10, BC>24.81357858, LAC>4.285714286, CC>0.523255814, EC>0.1152470335, NC>5.1101190475. The second screening result was 15 nodes and 68 edges, and the screened genes included MYC, AKT1, MAPK1, NFKBIA, MAPK8, RELA, FOS, EGFR, JUN, MAPK14, RB1, ESR1, TP53, CCND1, and CDKN1A. The final screening results are shown in FIG. 3.

### 2.5 Enrichment analysis of GO and KEGG pathways

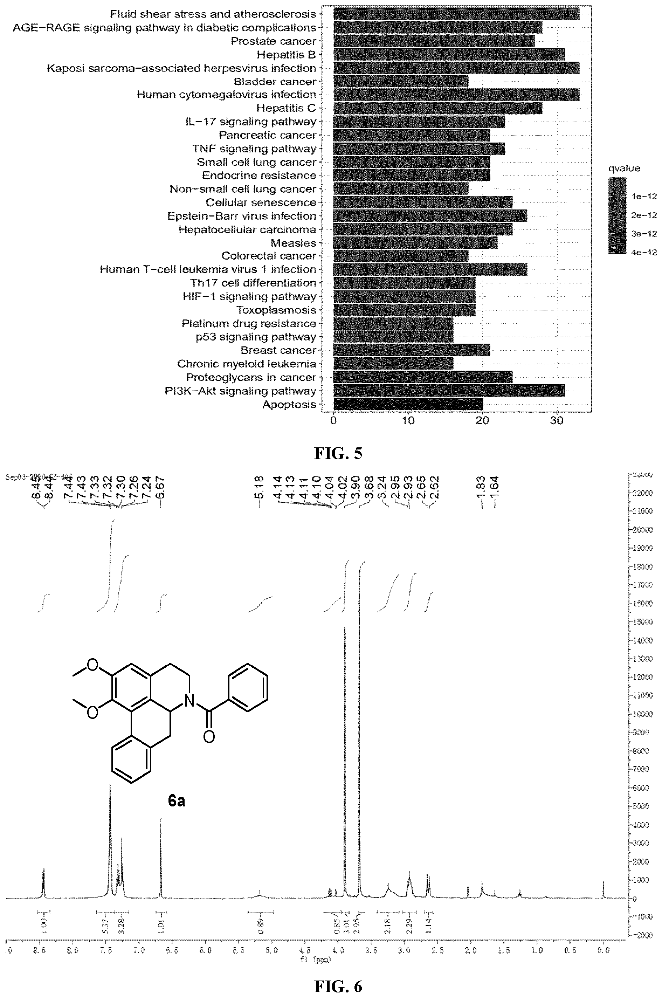

MF, BP and CC of 171 core indicators were analyzed. The results show that the target sites of lotus leaves are highly enriched in biological functions and processes: such as response to nutrient levels, response to lipopolysaccharide, response to molecule of bacterial origin, response to oxygen levels, cellular response to drug, cellular response to chemical stress, response to metal ion, and response to hypoxia. The first 20 GO analysis results were screened out, with P<0.05 as the threshold. The results are shown in FIG. 4. KEGG enrichment analysis was performed on 171 lotus leaf targets. The results show that the target proteins were enriched in 112 pathways, including fluid shear stress and atherosclerosis; Kaposi sarcoma-associated herpes virus infection; human cytomegalovirus infection; and the PI3K/AKT signaling pathway. These classical signaling pathways play an important role in the occurrence and development of obesity. The top 20 KEGG analysis results were screened out, with P<0.05 as the threshold, as shown in FIG. 5. GO and KEGG analyses indicate that lotus leaf could act on obesity-related targets through multiple pathways.

### 2.6 Analysis of molecular docking results

Based on nuciferine, the effective component of lotus leaf, the structure of nuciferine was modified. The modification results are shown below. Molecular docking was performed by using Autodock vina 1.5.6. The docking parameter setting and corresponding calculation results are shown in Table 2. The docking results show that benzoylnuciferine has good binding force with most related target proteins, which may be active components with the highest predicted target Degree among several modified structures of nuciferine.

The structural formulas and numbering of nuciferine and its modifications are as follows:

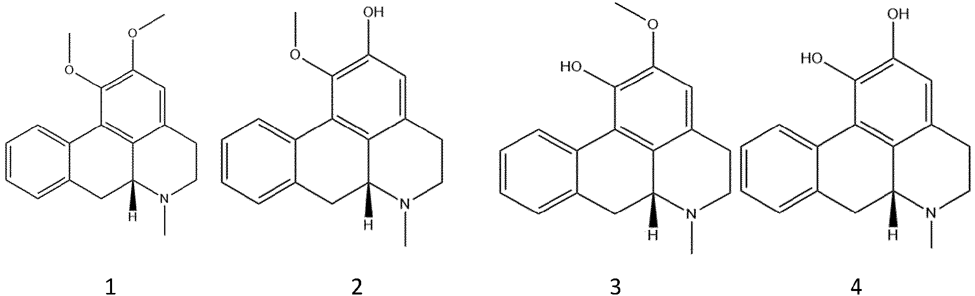

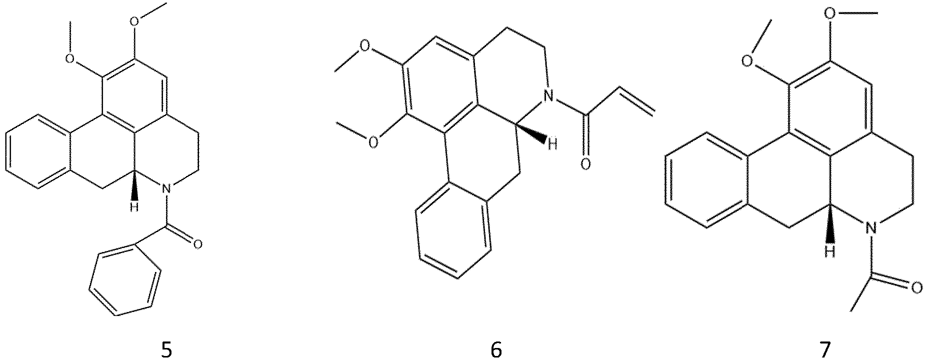

The predictions of binding degree of nuciferine and its modifications to obesity-related target proteins are shown in Table 2.

**Table 2 Binding degree of nuciferine and its modifications to obesity-related target proteins**

| Targets | 1 | 2 | 3 | 4 | 5 | 6 | 7 |
|---|---|---|---|---|---|---|---|
| ADRA2B | -7.7 | -7.8 | -7.6 | -7.8 | -0.8 | -5.3 | -6.2 |
| HSP90AB1 | -8.5 | -8.9 | -8.4 | -8.8 | -10.2 | -8.9 | -9.3 |
| RXRA | -8.1 | -8.5 | -8.8 | -8.6 | -7.2 | -8.4 | -8.9 |
| MYC | -7.3 | -7.6 | -8.1 | -7.8 | -3.1 | -7.1 | -7.7 |
| MAPK1 | -8.6 | -8.4 | -8.7 | -9 | -9.1 | -8.9 | -9.1 |
| NFKBIA | -7.6 | -7.9 | -8.4 | -9.2 | -9.4 | -8.1 | -8.6 |
| MAPK8 | -8.7 | -8.7 | -9.2 | -9.3 | -9.7 | -8.5 | -8.6 |
| FOS | -4.8 | -5.3 | -5.9 | -6 | -6.1 | -5.2 | -5.1 |
| JUN | -5.5 | -5.8 | -5.5 | -5.8 | -6.7 | -5.7 | -5.7 |
| RB1 | -5.4 | -5.5 | -5.6 | -5.7 | -5.7 | -5.6 | -5.4 |
| ESR1_5dxe | -8.3 | -8.6 | -8.3 | -8.8 | -7.7 | -8.7 | -8.5 |
| ESR1_4aa6 | -6.2 | -6.5 | -6.2 | -7.3 | -7.2 | -6.9 | -6.7 |
| TP53 | -6.3 | -6.3 | -6.3 | -6.3 | -6.7 | -6.3 | -6.6 |
| CCND1 | -5.3 | -5.7 | -6.5 | -6.4 | -6.5 | -5.4 | -5.3 |
| CDKN1A_6p8h | -6.2 | -6.3 | -6.4 | -6.4 | -7.1 | -6.5 | -6.2 |
| CDKN1A_5e0u | -5.5 | -5.4 | -5.6 | -5.5 | -6.8 | -5.5 | -5.8 |
| AKT1 | -10.2 | -10.4 | -10.6 | -10.7 | -12.1 | -10.2 | -10.9 |
| RELA | -6.6 | -6.5 | -7.5 | -7.7 | -7.6 | -6.6 | -6.9 |
| EGFR_5xwd | -6.4 | -7.2 | -7 | -7.6 | -7.2 | -6.8 | -6.9 |
| EGFR_3gop | -6.3 | -6.7 | -6.6 | -7.2 | -7.8 | -7.2 | -6.9 |
| MAPK14 | -8.2 | -8.5 | -8.6 | -8.8 | -8.3 | -9 | -9 |

### Example 2

### Preparation of benzoylnuciferine (denoted as compound 6a)

The synthesis route of the benzoylnuciferine was shown in scheme 1:

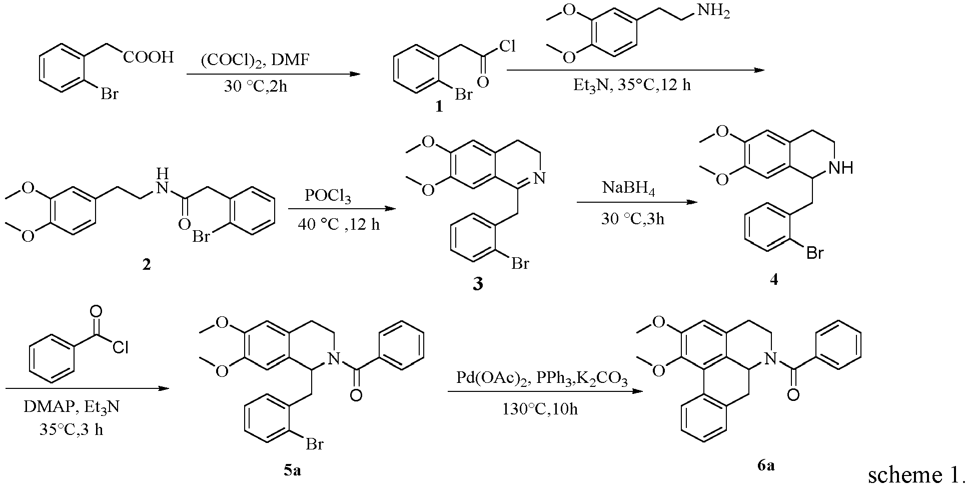

At 0 °C, oxalyl chloride (30 mmol, 2.54 mL) was added to 2-bromophenylacetic acid (30 mmol, 6.45 g), which had been dissolved in dichloromethane (DCM, 30 mL), and 0.6 g dimethylformamide (DMF) was then added thereto. The resulting mixture was reacted at 30 °C for 2 h. After the reaction, the resulting reaction system was subjected to rotary evaporation at 35 °C, to obtain Compound **1** (10.3 g, 92%).

3,4-dimethoxyphenethylamine (30 mmol, 5.06 mL) and triethylamine (33 mmol, 4.58 mL) were dissolved in DCM (40 mL), and compound **1** (20 mmol, 10 g) was then added thereto at 0 °C. The resulting mixture was reacted at 35 °C for 12 h. After the reaction, the reaction was quenched with 40 mL of brine, and the resulting reaction system was subjected to extraction with 100 mL of DCM. The resulting extraction phase was dried with 10 g of anhydrous sodium sulfate, and anhydrous sodium sulfate therein was removed by filtration. The resulting liquid phase was subjected to rotary evaporation at 35 °C, to obtain compound **2** (11.1 g, 98%). The ¹H-NMR data of compound **2** are as follows: ¹H NMR (500 MHz, CDCl₃) δ 7.53 (t, *J* = 7.3 Hz, 1H), 7.26 (dd, *J* = 6.4, 3.6 Hz, 2H), 7.17-7.10 (m, 1H), 6.71 (dd, *J* = 8.0, 4.9 Hz, 1H), 6.62 (s, 1H), 6.59 (dd, *J* = 8.0, 2.0 Hz, 1H), 5.47 (d, *J* = 27.9 Hz, 1H), 3.84 (d, *J* = 6.7 Hz, 3H), 3.82 (d, *J* = 6.5 Hz, 3H), 3.65 (d, *J =* 7.3 Hz, 2H), 3.50-3.41 (m, 2H), 2.74-2.64 (m, 2H).

Compound **2** (29 mmol, 11 g) was dissolved in DCM (60 mL), and POCl₃ (116 mmol, 10.81 mL) was then slowly dropped thereto. After dropping, a resulting mixture was refluxed at 40 °C for 12 h. After the reaction, the reaction was quenched by adding 45 mL of water thereto at 0 °C. After quenching, the resulting reaction system was subjected to extraction with 100 mL of DCM, and the resulting extraction phase was dried with 10 g of anhydrous sodium sulfate, followed by filtration; and the resulting liquid phase was subjected to rotary evaporation at 35 °C, to obtain compound **3** (10 g, 95.9%).

Compound **3** (27.8 mmol, 10 g) was dissolved in methanol (60 mL) and sodium borohydride (37.7 mmol, 1.43 g) was added thereto. The resulting mixture was reacted at 30 °C for 3 h. After the reaction was completed, the reaction was quenched by adding 30 mL of water thereto. The resulting reaction system after quenching was concentrated by rotary evaporation to remove a large amount of methanol therein, and then subjected to extraction with 40 mL of DCM. The resulting extraction phase was dried with 4 g of anhydrous sodium sulfate, and anhydrous sodium sulfate was then removed by filtration. The resulting liquid phase was subjected to rotary evaporation at 35 °C to obtain compound **4** (9.53 g, 95%). The ¹H-NMR data of compound **4** are as follows: ¹H-NMR (500 MHz, DMSO) δ 7.62 (d, *J =* 7.9 Hz, 1H), 7.40 (dd, *J =* 7.6, 1.4 Hz, 1H), 7.34 (t, *J=* 7.3 Hz, 1H), 7.23-7.16 (m, 1H), 6.68 (s, 1H), 6.61 (s, 1H), 4.16 (dd, *J=* 9.3, 4.3 Hz, 1H), 3.72 (s, 3H), 3.63 (s, 3H), 3.23 (dd, *J* = 13.6, 4.4 Hz, 1H), 3.20-3.13 (m, 1H), 3.00 (dd, J = 13.5, 9.6 Hz, 1H), 2.92-2.85 (m, 1H), 2.67 (q, *J* = 7.8 Hz, 2H).

Compound **4** (26.2 mmol, 9.5 g) and DMAP (5 mol%) were dissolved in DCM (30 mL), triethylamine (39.3 mmol, 5.45 mL) was then added thereto, and benzoyl chloride (31.5 mmol, 4.43 g) was dropped thereto at 0 °C. After the addition, a resulting mixture was reacted at 35 °C for 3 h. After the reaction was completed, the reaction was quenched by adding 30 mL of water thereto, and the resulting reaction system after quenching was subjected to extraction with 60 mL of DCM. The resulting extraction phase was dried with 10 g of anhydrous sodium sulfate, and anhydrous sodium sulfate was then removed by filtration. The resulting liquid phase was subjected to rotary evaporation at 35 °C to obtain compound **5a** (11.6 g, 94.9%).

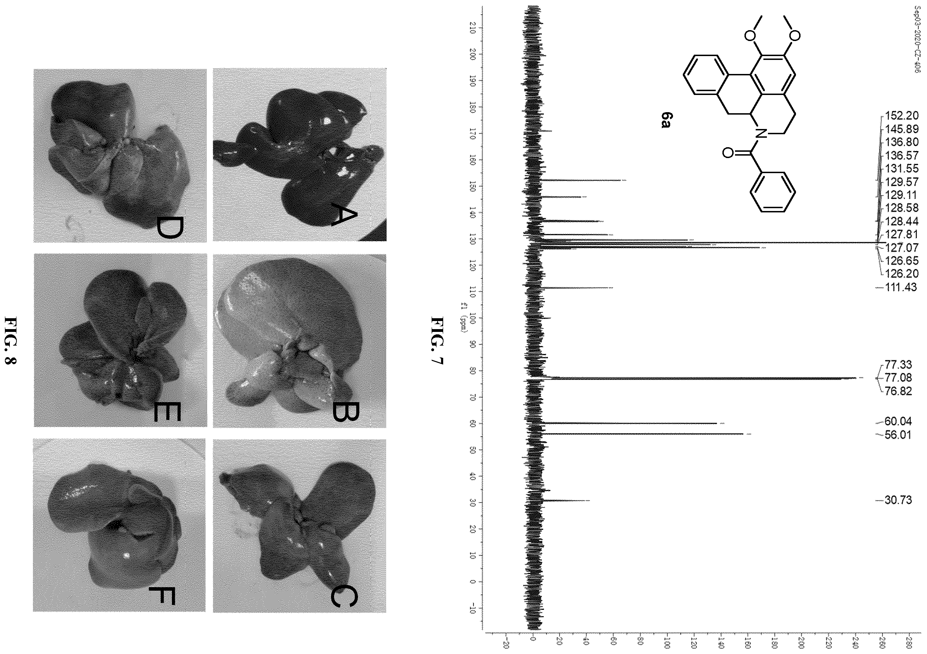

Compounds **5a** (10 mmol, 4.65 g), Pd(OAc)₂ (5 mol%, 112 mg), PPh₃ (20 mol%, 520 mg), K₂CO₃ (20 mmol, 2.760 g) were added into Young's tube, and N₂ was introduced therein. 40 mL of N,N-dimethylacetamide was added dropwise thereto. The resulting mixture was reacted at 130 °C for 10 h. After the reaction was completed, the reaction was quenched by adding 40 mL of water thereto, and the resulting reaction system after quenching was subjected to extraction with 80 mL of DCM. The resulting extraction phase was dried with 15 g of anhydrous sodium sulfate, and anhydrous sodium sulfate was then removed by filtration. The resulting liquid phase was subjected to rotary evaporation at 35 °C to obtain compound 6a (3.4g, 88.2%). The ¹H-NMR of compound **6a** is shown in FIG. 6, and the ¹³C-NMR is shown in FIG. 7. The specific nuclear magnetic detection data are as follows: ¹H NMR (500 MHz, CDCl₃) δ 8.47 (d, *J =* 7.8 Hz, 1H), 7.46 (d, *J =* 3.3 Hz, 6H), 7.39-7.22 (m, 4H), 6.70 (s, 1H), 5.21 (s, 1H), 4.11 (dt, *J =* 39.5, 8.3 Hz, 1H), 3.92 (s, 3H), 3.70 (s, 3H), 3.27 (s, 2H), 2.96 (d, *J =* 13.0 Hz, 2H), 2.66 (d, *J =* 15.3 Hz, 1H); ¹³C NMR (126 MHz, CDCl₃) δ 152.20, 145.89, 136.80, 136.57, 131.55, 129.57, 129.11, 128.58, 128.44, 127.81, 127.07, 126.65, 126.20, 111.43, 77.33, 77.08, 76.82, 60.04, 56.01, 30.73.

### Example 3

p-Fluorobenzoylnuciferine (denoted as compound **6b)** was prepared, which had a structural formula as follows:

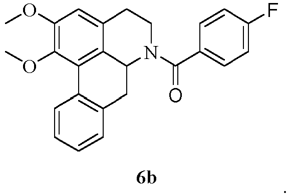

At 0 °C, phosphorus trichloride (45 mmol, 6.18 g) was added to 2-bromophenylacetic acid (45 mmol, 9.68 g), which had been dissolved in chloroform (45 mL). 0.8 g of DMF was added thereto. The resulting mixture was reacted at 20 °C for 5 h. After the reaction, the resulting reaction system was subjected to rotary evaporation at 35 °C, to obtain Compound **1** (15.4 g, 91.6%).

3,4-dimethoxyphenethylamine (45 mmol, 7.59 mL) and pyridine (49.5 mmol, 3.92 g) were dissolved in chloroform (60 mL), and compound **1** (30 mmol, 15 g) was then added thereto at 0 °C. The resulting mixture was reacted at 30 °C for 10 h. After the reaction was completed, the reaction was quenched with 60 mL of brine. The reaction resulting system after quenching was subjected to extraction with 150 mL of DCM. The resulting extraction phase was dried with 12 g of anhydrous sodium sulfate, and anhydrous sodium sulfate was then removed by filtration. The resulting liquid phase was subjected to rotary evaporation at 35 °C to obtain compound **2** (16 g, 97%).

Compound **2** (29 mmol, 11 g) was dissolved in chloroform (60 mL), and POCl₃ (116 mmol, 10.81 mL) was slowly added dropwise thereto. After the addition, a resulting mixture was refluxed at 40 °C for 12 h. After the reaction was completed, the reaction was quenched by adding 45 mL of water thereto at 0 °C. After quenching, a resulting system was subjected to extraction with 100 mL of DCM, and a resulting extraction phase was dried with 10 g of anhydrous sodium sulfate, followed by filtration; and a resulting liquid phase was subjected to rotary evaporation at 35 °C, to obtain compound **3** (9.8g, 94%).

Compound **3** (27.8 mmol, 10 g) was dissolved in ethanol (60 mL), and potassium borohydride (35 mmol, 1.89 g) was added thereto. The resulting mixture was reacted at 40 °C for 2.5 h. After the reaction was completed, the reaction was quenched by adding 30 mL of water thereto. The resulting reaction system after quenching was concentrated by rotary evaporation to remove a large amount of ethanol therein, and then subjected to extraction with 40 mL of DCM. The resulting extraction phase was dried with 4 g of anhydrous sodium sulfate, and anhydrous sodium sulfate was then removed by filtration. The resulting liquid phase was subjected to rotary evaporation at 35 °C to obtain compound **4** (9.5 g, 94.7%).

Compound **4** (39.5 mmol, 14.3 g) was dissolved in ethanol (50 mL), and NaOH (59 mmol, 8.2 g) was then added thereto. At 0 °C, p-fluorobenzoyl chloride (47.6 mmol, 7.53 g) was added dropwise thereto. After the addition, a resulting mixture was reacted at 40 °C for 2 h. After the reaction was completed, the reaction was quenched by adding 50 mL of water thereto. The resulting reaction system was subjected to extraction with 80 mL of DCM, and a resulting extraction phase was dried with 10 g of anhydrous magnesium sulfate. The anhydrous magnesium sulfate was then removed by filtration, and a resulting liquid phase was subjected to rotary evaporation at 35 °C to obtain compound **5b** (17.2 g, 90%).

Compound **5b** (15 mmol, 7.26 g), nickel chloride (6 mol%, 116.6 mg), PBu₃ (15 mol%, 455 mg), Na₂CO₃ (30 mmol, 3.17 g) were added into Young's tube, and N₂ was introduced thereto. 60 mL of N,N-dimethylformamide was added dropwise thereto. The resulting mixture was reacted at 120 °C for 8 h. After the reaction was completed, the reaction was quenched by adding 60 mL of water thereto. The resulting reaction system after quenching was subjected to extraction with 100 mL of DCM, and a resulting extraction phase was dried with 10 g of anhydrous magnesium sulfate. The anhydrous magnesium sulfate was then removed by filtration, and a resulting liquid phase was subjected to rotary evaporation at 35 °C to obtain compound **6b** (5.2 g, 86%). The ¹H-NMR data of compound **6b** are as follows: ¹H NMR (500 MHz, CDCl₃) δ 8.44 (d, *J* = 7.9 Hz, 1H), 7.46 (dd, *J* = 8.5, 5.4 Hz, 2H), 7.35-7.21 (m, 4H), 7.12 (t, *J* = 8.5 Hz, 2H), 6.67 (s, 1H), 5.13 (s, 1H), 4.05 (d, *J* = 1.5 Hz, 1H), 3.90 (s, 3H), 3.68 (s, 3H), 3.20 (d, *J* = 66.4 Hz, 2H), 2.93 (t, *J=* 16.1 Hz, 2H), 2.66 (d, *J=* 15.4 Hz, 1H).

### Example 4

p-Methylbenzoylnuciferine (denoted as compound **6c)** was prepared, which had a structural formula as follows:

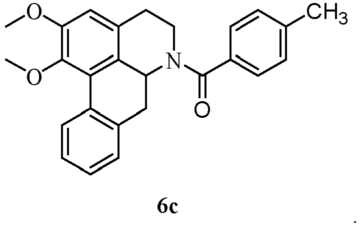

At 0 °C, phosphorus pentachloride (30 mmol, 6.25 g) was added to 2-bromophenylacetic acid (30 mmol, 6.45 g), which had been dissolved in ethanol (30 mL), and 0.6 g of ferric chloride was then added thereto. The resulting mixture was reacted at 35 °C for 3 h. After the reaction was completed, the resulting reaction system was subjected to rotary evaporation at 35 °C, to obtain Compound **1** (10 g, 90.2%).

3,4-dimethoxyphenethylamine (15 mmol, 2.53 mL) and potassium carbonate (16.5 mmol, 2.28 g) were dissolved in ethanol (20 mL), and at 0 °C, compound **1** (10 mmol, 5 g) was then added thereto. The resulting mixture was reacted at 35 °C for 10 h. After the reaction was completed, the reaction was quenched by adding 20 mL of brine thereto, and the resulting reaction system was subjected to extraction with 50 mL of DCM. The resulting extraction phase was dried with 5 g of anhydrous sodium sulfate, and anhydrous sodium sulfate was then removed by filtration. The resulting liquid phase was subjected to rotary evaporation at 35 °C to obtain compound **2** (5.5 g, 97%).

Compound **2** (14.5 mmol, 5.5 g) was dissolved in ethanol (30 mL), and POCl₃ (58 mmol, 5.4 g) was then slowly added dropwise thereto. After the addition, a resulting mixture was refluxed at 44 °C for 13 h. After the reaction was completed, the reaction was quenched by adding 20 mL of water at 0 °C. After quenching, the resulting reaction system was subjected to extraction with 50 mL of DCM, and a resulting extraction phase was dried with 5 g of anhydrous sodium sulfate, followed by filtration. The resulting liquid phase was subjected to rotary evaporation at 35 °C, obtaining compound **3** (4.9 g, 95%).

Compound **3** (27.8 mmol, 10 g) was dissolved in methanol (60 mL), and sodium borohydride (37.7 mmol, 1.43 g) was then added thereto. The resulting mixture was reacted at 30 °C for 3 h. After the reaction was completed, the reaction was quenched by adding 30 mL of water thereto. The resulting reaction system after quenching was concentrated by rotary evaporation to remove a large amount of methanol therein, and then subjected to extraction with 40 mL of DCM. The resulting extraction phase was dried with 4 g of anhydrous sodium sulfate, and anhydrous sodium sulfate was then removed by filtration. The resulting liquid phase was subjected to rotary evaporation at 35 °C to obtain compound **4** (9.53 g, 95%).

Compound **4** (13.2 mmol, 4.77 g) was dissolved in methanol (15 mL), and KOH (20 mmol, 1.2 g) was then added thereto. At 0 °C, p-methylbenzoyl chloride (15.8 mmol, 7.53 g) was added dropwise thereto, and the resulting mixture was reacted at 40 °C for 3 h. After the reaction was completed, the reaction was quenched by adding 50 mL of water thereto, and the resulting reaction system was subjected to extraction with 20 mL of DCM. The resulting extraction phase was dried with 2 g of solid calcium oxide, and the solid calcium oxide was then removed by filtration. The resulting liquid phase was subjected to rotary evaporation at 35 °C to obtain compound **5c** (5.6 g, 88.6%).

Compound **5c** (5 mmol, 2.4 g), bis(triphenylphosphine)nickel chloride (5 mol%, 164.04 mg), t-PBu₃ (15 mol%, 81 mg), NaHCO₃ (10 mmol, 0.84 g) were added into Young's tube and N₂ was introduced thereto. 20 mL of tetrahydrofuran was added dropwise thereto, and the resulting mixture was reacted at 140 °C for 10 h. After the reaction was completed, the reaction was quenched by adding 20 mL of water thereto, and the resulting system was subjected to extraction with 40 mL of DCM. The resulting extraction phase was dried with 2 g of solid calcium oxide, and the solid calcium oxide was then removed by filtration. The resulting liquid phase was subjected to rotary evaporation at 35 °C to obtain compound **6c** (1.8 g, 90%). The ¹H-NMR and ¹³C-NMR data of compound **6c** are as follows: ¹H NMR (500 MHz, CDCl₃) δ 8.43 (d, *J =* 7.8 Hz, 1H), 7.42-7.15 (m, 8H), 6.66 (s, 1H), 5.14 (s, 1H), 4.11 (dd, *J =* 14.3, 7.1 Hz, 1H), 3.89 (s, 3H), 3.68 (d, *J* = 6.6 Hz, 3H), 3.21 (dd, *J* = 33.4, 21.6 Hz, 2H), 2.91 (t, *J* = 13.7 Hz, 2H), 2.62 (d, *J =* 15.4 Hz, 1H), 2.38 (s, 3H); ¹³C NMR (126 MHz, CDCl₃) δ 152.29 (s), 148.19-144.39 (m), 139.77 (s), 136.77 (s), 134.01 (s), 131.69 (s), 129.27 (s), 128.60 (d, *J =* 12.2 Hz), 127.90 (s), 127.15 (s), 126.88 (s), 126.57-126.35 (m), 111.52 (s), 77.41 (s), 77.16 (s), 76.91 (s), 60.16 (s), 56.13 (s), 30.89 (s), 21.53 (s), 2.63--2.96 (m).

### Example 5

o-Methylbenzoylnuciferine (denoted as compound **6d)** was prepared, which had a structural formula as follows:

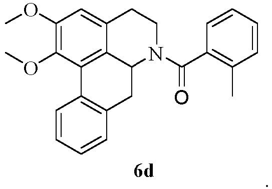

At 0 °C, sulfone chloride (36 mmol, 4.28 g) was added to 2-bromophenylacetic acid (36 mmol, 7.74 g), which had been dissolved in methanol (36 mL), and 0.72 g of imidazole was then added thereto. The resulting mixture was reacted at 45 °C for 5 h. After the reaction was completed, the resulting reaction system was subjected to rotary evaporation at 35 °C, to obtain Compound **1** (12.36 g, 92.1%).

3,4-dimethoxyphenethylamine (36 mmol, 6.07 mL) and triethylamine (39.6 mmol, 5.496 mL) were dissolved in DCM (48 mL), and at 0 °C, compound **1** (24 mmol, 12 g) was then added thereto. The resulting mixture was reacted at 35 °C for 13 h. After the reaction was completed, the reaction was quenched by adding 50 mL of brine thereto, and the resulting reaction system was subjected to extraction with 100 mL of DCM. The extraction phase was dried with 10 g of anhydrous sodium sulfate, and anhydrous sodium sulfate was then removed by filtration. The resulting liquid phase was subjected to rotary evaporation at 35 °C to obtain compound **2** (13.32 g, 97.9%).

Compound **2** (29 mmol, 11 g) was dissolved in DCM (60 mL), and then POCl₃ (116 mmol, 10.81 mL) was then slowly added dropwise thereto. After the addition, the resulting mixture was refluxed at 40 °C for 12 h. After the reaction was completed, the reaction was quenched by adding 45 mL of water thereto at 0 °C. The resulting reaction system after the quenching was subjected to extraction with 100 mL of DCM. The resulting extraction phase was dried with 10 g of anhydrous sodium sulfate, followed by filtration. The resulting liquid phase was subjected to rotary evaporation at 35 °C, to obtain compound **3** (10 g, 95.9%).

Compound **3** (27.8 mmol, 10 g) was dissolved in methanol (60 mL) and potassium borohydride (37.7 mmol, 2.03 g) was added thereto. The resulting mixture was reacted at 30 °C for 3 h. After the reaction was completed, the reaction was quenched by adding 30 mL of water thereto. The resulting reaction system after quenching was concentrated by rotary evaporation to remove a large amount of methanol therein, and then subjected to extraction with 40 mL of DCM. The resulting extraction phase was dried with 4 g of anhydrous sodium sulfate, and anhydrous sodium sulfate was then removed by filtration. The resulting liquid phase was subjected to rotary evaporation at 35 °C to obtain compound **4** (9.53 g, 95%).

Compound **4** (52.8 mmol, 19.1 g) was dissolved in trichloromethane (60 mL), and potassium tert-butoxide (80 mmol, 8.98 g) was added thereto, and at 0 °C, o-methylbenzoyl chloride (63.28 mmol, 9.78 g) was added dropwise thereto. After the addition, the resulting mixture was reacted at 25 °C for 4 h. After the reaction was completed, the reaction was quenched by adding 60 mL of water thereto, and the resulting reaction system after quenching was subjected to extraction with 60 mL of DCM. The resulting extraction phase was dried with 6 g of anhydrous sodium sulfate, and anhydrous sodium sulfate was then removed by filtration. The resulting liquid phase was subjected to rotary evaporation at 35 °C to obtain compound **5d** (21.7 g, 85.5%).

Compound **5d** (20 mmol, 9.6 g), palladium on carbon (10 mol%, 212.84 mg), PPh₃ (30 mol%, 1.6 g) and NaOH (40 mmol, 0.16 g) were added into Young's tube, N₂ was introduced thereto, and 80 mL of methylpyrrolidone was added dropwise thereto. The resulting mixture was reacted at 150 °C for 9 h. After the reaction was completed, the reaction was quenched by adding 50 mL of water thereto, and 80 mL of DCM was added for extraction. The resulting extraction phase was dried with 8 g of anhydrous sodium sulfate, and anhydrous sodium sulfate was then removed by filtration. The resulting liquid phase was subjected to rotary evaporation at 35 °C to obtain compound **6d** (7.2 g, 90.1%). The ¹H-NMR and ¹³C-NMR data of compound **6d** are as follows: ¹H NMR (500 MHz, CDCl₃) δ 8.38 (d, *J* = 7.8 Hz, 1H), 7.32-7.07 (m, 8H), 6.57 (s, 1H), 5.22 (dd, *J* = 13.7, 3.7 Hz, 1H), 4.04 (q, *J* = 7.1 Hz, 1H), 3.81 (s, 3H), 3.67-3.50 (m, 4H), 3.16 (ddd, *J =* 14.6, 13.1, 3.0 Hz, 2H), 2.91-2.67 (m, 2H), 2.50 (dd, *J=* 25.2, 15.8 Hz, 1H), 2.27 (d, *J* = 31.2 Hz, 3H). ¹³C NMR (126 MHz, CDCl₃) δ 152.18 (s), 145.93 (s), 136.54 (s), 134.22 (s), 131.58 (s), 130.32 (s), 128.56 (dt, *J* = 67.7, 36.3 Hz), 127.92 (s), 127.86 (d, *J =* 15.1 Hz), 127.06 (s), 126.41-126.23 (m), 126.01 (d, *J =* 47.1 Hz), 125.25 (s), 111.42 (s), 77.31 (s), 77.06 (s), 76.80 (s), 60.04 (s), 56.00 (s), 50.41 (s), 41.98 (s), 33.97 (s), 30.61 (s), 18.79 (s).

### Example 6

p-Methoxybenzoylnuciferine (denoted as compound **6e)** was prepared, which had a structural formula as follows:

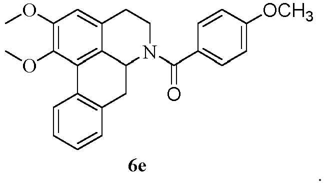

At 0 °C, sulfoxide chloride (30 mmol, 3.57 g) was added to 2-bromophenylacetic acid (30 mmol, 6.45 g), which had been dissolved in acetonitrile (30 mL), and 0.6 g of aluminum trichloride was then added thereto. The resulting mixture was reacted at 45 °C for 2 h. After the reaction was completed, the resulting reaction system was subjected to rotary evaporation at 35 °C, to obtain Compound **1** (10.3 g, 92%).

3,4-dimethoxyphenethylamine (30 mmol, 5.06 mL) and triethylamine (33 mmol, 4.58 mL) were dissolved in DCM (40 mL), and compound 1 (20 mmol, 10 g) was then added thereto at 0 °C. The resulting mixture was reacted at 35 °C for 12 h. After the reaction was completed, the reaction was quenched by adding 40 mL of brine thereto, and the resulting reaction system was subjected to extraction with 100 mL of DCM. The resulting extraction phase was dried with 10 g of anhydrous sodium sulfate, and anhydrous sodium sulfate was then removed by filtration. The resulting liquid phase was subjected to rotary evaporation at 35 °C to obtain compound **2** (11.1 g, 98%).

Compound **2** (29 mmol, 11 g) was dissolved in DCM (60 mL), and POCl₃ (116 mmol, 10.81 mL) was then slowly added dropwise thereto. After the addition, the resulting mixture was refluxed at 40 °C for 12 h. After the reaction was completed, the reaction was quenched by adding 45 mL of water thereto at 0 °C. After quenching, the resulting reaction system was subjected to extraction with 100 mL of DCM, and the resulting extraction phase was dried with 10 g of anhydrous sodium sulfate, followed by filtration. The resulting liquid phase was subjected to rotary evaporation at 35 °C, to obtain compound **3** (10 g, 95.9%).

Compound **3** (27.8 mmol, 10 g) was dissolved in methanol (60 mL) and sodium borohydride (37.7 mmol, 1.43 g) was added thereto. The resulting mixture was reacted at 30 °C for 3 h. After the reaction was completed, the reaction was quenched by adding 30 mL of water thereto. The resulting reaction system was concentrated by rotary evaporation to remove a large amount of methanol therein, and then subjected to extraction with 40 mL of DCM. The resulting extraction phase was dried with 4 g of anhydrous sodium sulfate, and anhydrous sodium sulfate was then removed by filtration. The resulting liquid phase was subjected to rotary evaporation at 35 °C to obtain compound **4** (9.53 g, 95%).

Compound **4** (13.2 mmol, 4.77 g) was dissolved in dichloromethane (15 mL), ammonium acetate (20 mmol, 1.5 g) was then added thereto, and p-methoxybenzoyl chloride (15.82 mmol, 2.7 g) was added dropwise thereto at 0 °C. After the addition, the resulting mixture was reacted at 30 °C for 3 h. After the reaction was completed, the reaction was quenched by adding 50 mL of water thereto, and the resulting reaction system after quenching was subjected to extraction with 20 mL of DCM. The resulting extraction phase was dried with 2 g of solid calcium oxide, and the solid calcium oxide was removed by filtration. The resulting liquid phase was subjected to rotary evaporation at 35 °C to obtain compound **5e** (5.9 g, 90.1%).

Compound **5e** (5 mmol, 2.5 g), palladium chloride (5 mol%, 44.3 mg), PPh₃ (15 mol%, 200 mg), K₂CO₃ (10 mmol, 1.38 g) were added into Young's tube, N₂ was introduced thereto, and 20 mL of N,N-dimethylacetamide was added dropwise thereto. The resulting mixture was reacted at 150 °C for 8 h. After the reaction was completed, the reaction was quenched by adding 20 mL of water thereto, and 40 mL of DCM was added for extraction. The resulting extraction phase was dried with 2 g of solid calcium oxide, and the solid calcium oxide was then removed by filtration. The resulting liquid phase was subjected to rotary evaporation at 35 °C, to obtain compound **6e** (1.7 g, 81.8%). The ¹H-NMR and ¹³C-NMR data of compound **6e** are as follows: ¹H NMR (500 MHz, CDCl₃) δ 8.39 (d, *J* = 7.8 Hz, 1H), 7.38 (d, *J* = 8.6 Hz, 2H), 7.29-7.14 (m, 4H), 6.89 (d, *J* = 8.6 Hz, 2H), 6.63 (s, 1H), 5.09 (s, 1H), 4.18 (s, 1H), 3.86 (s, 3H), 3.79 (s, 3H), 3.63 (s, 3H), 3.21 (t, *J* = 12.3 Hz, 1H), 3.09 (d, *J =* 11.3 Hz, 1H), 2.87 (t, *J* = 13.7 Hz, 2H), 2.60 (d, *J* = 15.4 Hz, 1H). ¹³C NMR (126 MHz, CDCl₃) δ 160.63 (s), 152.16 (s), 146.25-145.27 (m), 136.66 (s), 131.56 (s), 129.20-129.07 (m), 128.99-128.93 (m), 128.67 (s), 128.46 (d, *J* = 10.0 Hz), 127.78 (s), 127.03 (s), 126.44-126.35 (m), 126.52-126.09 (m), 113.80 (s), 111.38 (s), 77.28 (s), 77.02 (s), 76.77 (s), 60.03 (s), 56.00 (s), 55.36 (s), 30.78 (s).

### Example 7

Acetylnuciferine (denoted as compound **6f)** was prepared, which had a structural formula as follows:

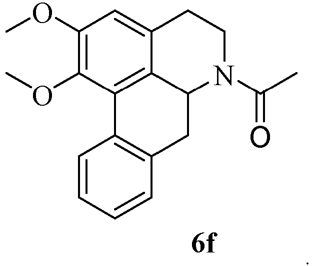

At 0 °C, triphosgene (30 mmol, 8.9 g) was added to 2-bromophenylacetic acid (30 mmol, 6.45 g), which had been dissolved in tetrahydrofuran (30 mL), and 0.6 g of DMF was added thereto. The resulting mixture was reacted at 25 °C for 3 h. After the reaction was completed, the resulting reaction system was subjected to rotary evaporation at 35 °C, to obtain Compound **1** (10.3 g, 92%).

3,4-dimethoxyphenethylamine (30 mmol, 5.06 mL) and triethylamine (33 mmol, 4.58 mL) were dissolved in DCM (40 mL), and compound **1** (20 mmol, 10 g) was added thereto at 0 °C. The resulting mixture was reacted at 35 °C for 12 h. After the reaction was completed, the reaction was quenched by adding 40 mL of brine thereto, and the resulting reaction system was subjected to extraction with 100 mL of DCM. The resulting extraction phase was dried with 10 g of anhydrous sodium sulfate, and anhydrous sodium sulfate was then removed by filtration. The resulting liquid phase was subjected to rotary evaporation at 35 °C to obtain compound **2** (11.1 g, 98%).

Compound **2** (29 mmol, 11 g) was dissolved in DCM (60 mL), and POCl₃ (116 mmol, 10.81 mL) was then slowly added dropwise thereto. After the addition, the resulting mixture was refluxed at 40 °C for 12 h. After the reaction was completed, the reaction was quenched by adding 45 mL of water thereto at 0 °C. After quenching, the resulting reaction system was subjected to extraction with 100 mL of DCM. The resulting extraction phase was dried with 10 g of anhydrous sodium sulfate, followed by filtration. The resulting liquid phase was subjected to rotary evaporation at 35 °C, to obtain compound **3** (10 g, 95.9%).

Compound **3** (27.8 mmol, 10 g) was dissolved in methanol (60 mL) and sodium borohydride (37.7 mmol, 1.43 g) was added thereto. The resulting mixture was reacted at 30 °C for 3 h. After the reaction was completed, the reaction was quenched by adding 30 mL of water thereto. The resulting reaction system was concentrated by rotary evaporation to remove a large amount of methanol therein, and then subjected to extraction with 40 mL of DCM. The resulting extraction phase was dried with 4 g of anhydrous sodium sulfate, and anhydrous sodium sulfate was then removed by filtration. The resulting liquid phase was subjected to rotary evaporation at 35 °C to obtain compound **4** (9.53 g, 95%).

Compound **4** (13.2 mmol, 4.77 g) and DMAP (6.6 mmol, 80.5 mg) were dissolved in dichloromethane (15 mL), triethylamine (19.8 mmol, 2.75 mL) was then added thereto, and at 0 °C, acetyl chloride (15.82 mmol, 1.12 mL) was added dropwise thereto. After the addition, the resulting mixture was reacted at 35 °C for 3 h. After the reaction was completed, the reaction was quenched by adding 50 mL of water thereto. The resulting reaction system after quenching was subjected to extraction with 20 mL of DCM. The resulting extraction phase was dried with 2 g of anhydrous sodium sulfate, and anhydrous sodium sulfate was then removed by filtration. The resulting liquid phase was subjected to rotary evaporation at 35 °C to obtain compound **5f** (4.8 g, 89.9%).

Compounds **5f (2** mmol, 1.6 g), tetra(triphenylphosphine)palladium (1 mol%, 23.1 mg), PPh₃ (4 mol%, 104 mg), K₂CO₃ (4 mmol, 552 mg) were added to Young's tube, N₂ was introduced thereto, and 10 mL of N,N-dimethylacetamide was added dropwise thereto. The resulting mixture was reacted at 130 °C for 12 h. After the reaction was completed, the reaction was quenched by adding 15 mL of water thereto. The resulting reaction system after quenching was subjected to extraction with 20 mL of DCM. The resulting extraction phase was dried with 2 g of anhydrous sodium sulfate, and anhydrous sodium sulfate was then removed by filtration. The resulting liquid phase was subjected to rotary evaporation at 35 °C to obtain compound 6f (0.63 g, 90%). The ¹H-NMR and ¹³C-NMR data of compound 6f are as follows: ¹H NMR (500 MHz, CDCl₃) δ 8.34 (dd, *J* = 22.2, 7.8 Hz, 1H), 7.30-7.10 (m, 4H), 6.58 (d, *J=* 18.0 Hz, 1H), 4.99 (d, *J* = 10.9 Hz, 1H), 4.86 (d, *J* = 11.6 Hz, 1H), 4.46 (d, *J* = 11.9 Hz, 1H), 3.94-3.85 (m, 1H), 3.80 (s, 3H), 3.56 (s, 3H), 3.21 (t, *J* = 12.1 Hz, 1H), 3.04-2.88 (m, 1H), 2.85-2.50 (m, 4H), 2.09 (d, *J =* 28.5 Hz, 3H). ¹³C NMR (126 MHz, CDCl₃) δ 152.05 (s), 136.78 (s), 128.60 (s), 128.38 (s), 127.75 (s), 126.94 (s), 111.31 (s), 100.89-99.31 (m), 77.30 (s), 77.04 (s), 76.79 (s), 60.01 (s), 55.99 (s), 53.82 (s), 50.37 (s), 41.96 (s), 36.42 (s), 34.03 (s), 30.76 (s), 22.62 (s).

### Example 8

Nuciferine (denoted as compound **6g)** was prepared, which had a structural formula as follows:

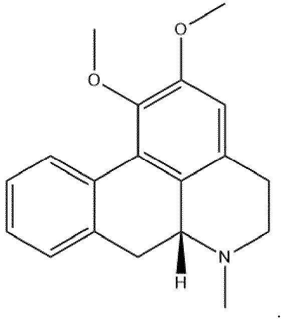

At 0 °C, oxalyl chloride (30 mmol, 2.54 mL) was added to 2-bromophenylacetic acid (30 mmol, 6.45 g), which had been dissolved in DCM (30 mL), and 0.6 g of DMF was then added thereto. The resulting mixture was reacted at 30 °C for 2 h. After the reaction was completed, the resulting reaction system was subjected to rotary evaporation at 35 °C, to obtain Compound 1 (10.3 g, 92%).

3,4-dimethoxyphenethylamine (30 mmol, 5.06 mL) and triethylamine (33 mmol, 4.58 mL) were dissolved in DCM (40 mL), and at 0 °C, compound 1 (20 mmol, 10 g) was added thereto. The resulting mixture was reacted at 35 °C for 12 h. After the reaction was completed, the reaction was quenched by adding 40 mL of brine thereto. The resulting reaction system after quenching was subjected to extraction with 100 mL of DCM. The resulting extraction phase was dried with 10 g of anhydrous sodium sulfate, and anhydrous sodium sulfate was then removed by filtration. The resulting liquid phase was subjected to rotary evaporation at 35 °C to obtain compound **2** (11.1 g, 98%).

Compound **2** (29 mmol, 11 g) was dissolved in DCM (60 mL), and POCl₃ (116 mmol, 10.81 mL) was slowly added dropwise thereto. After the addition, the resulting mixture was refluxed at 40 °C for 12 h. After the reaction was completed, the reaction was quenched by adding 45 mL of water thereto at 0 °C. The resulting reaction system after quenching was subjected to extraction with 100 mL of DCM, and the resulting extraction phase was dried with 10 g of anhydrous sodium sulfate, followed by filtration. The resulting liquid phase was subjected to rotary evaporation at 35 °C, to obtain compound **3** (10 g, 95.9%).

Compound **3** (27.8 mmol, 10 g) was dissolved in methanol (60 mL) and sodium borohydride (37.7 mmol, 1.43 g) was added thereto. The resulting mixture was reacted at 30 °C for 3 h. After the reaction was completed, the reaction was quenched by adding 30 mL of water thereto. The resulting reaction system after quenching was concentrated by rotary evaporation to remove a large amount of methanol therein, and then subjected to extraction with 40 mL of DCM. The resulting extraction phase was dried with 4 g of anhydrous sodium sulfate, and anhydrous sodium sulfate was then removed by filtration. The resulting liquid phase was subjected to rotary evaporation at 35 °C to obtain compound **4** (9.53 g, 95%).

Compound **4** (26.2 mmol, 9.5 g) and DMAP (5 mol%) were dissolved in DCM (30 mL), triethylamine (39.3 mmol, 5.45 mL) was then added thereto, and at 0 °C, iodomethane (31.5 mmol, 4.47 g) was added dropwise thereto. The resulting mixture was reacted at 35 °C for 3 h. After the reaction was completed, the reaction was quenched by adding 30 mL of water thereto, and the resulting reaction system after quenching was subjected to extraction with 60 mL of DCM. The resulting extraction phase was dried with 10 g of anhydrous sodium sulfate, and anhydrous sodium sulfate was then removed by filtration. The resulting liquid phase was subjected to rotary evaporation at 35 °C to obtain compound **5g** (9.6 g, 97.3%).

Compounds **5g** (10 mmol, 3.76 g), palladium on carbon (5 mol%, 112 mg), PPh₃ (20 mol%, 520 mg), and K₂CO₃ (20 mmol, 2.760 g) were added into Young's tube, N₂ was introduced thereto, and 40 mL of N,N-dimethylacetamide was added dropwise thereto. The resulting mixture was reacted at 130 °C for 10 h. After the reaction was completed, the reaction was quenched by adding 40 mL of water thereto, and the resulting reaction system after quenching was subjected to extraction with 80 mL of DCM. The resulting extraction phase was dried with 15 g of anhydrous sodium sulfate, and anhydrous sodium sulfate was then removed by filtration. The resulting liquid phase was subjected to rotary evaporation at 35 °C to obtain compound **6g** (2.6 g, 88%). ¹H NMR (500 MHz, CDCl₃) δ 8.39 (d, *J* = 7.8 Hz, 1H), 7.36-7.31 (m, 1H), 7.30-7.21 (m, 2H), 6.66 (s, 1H), 3.91 (s, 3H), 3.68 (s, 3H), 3.24-3.15 (m, 1H), 3.12 (dd, *J =* 13.5, 4.0 Hz, 1H), 3.07 (dd, *J =* 11.5, 6.1 Hz, 2H), 2.71 (dd, *J =* 16.2, 3.3 Hz, 1H), 2.65 (t, *J=* 13.5 Hz, 1H), 2.57 (s, 3H), 2.56-2.50 (m, 1H).

### Example 8

### In vivo experiment

### 1. Materials and methods

Animals: 54 SPF-grade SD rats, weighing 120±10 g, provided by Laboratory Animal Business Department, Shanghai Family Planning Research Institute, China, Animal license number: SCXK(Shanghai)2018-0006.

Drug to be tested: Nuciferine purchased from Shanghai Aladdin Biochemical Technology Co., Ltd., China, and its derivatives (purity >99%) that were synthesized in the laboratory by the methods of Examples 2-7.

Other reagents: AST, ALT, TC, TG, NEFA, Bun, Cr, CAT, GSH-Px, GSH, MDA test kits, purchased from Nanjing Jiancheng Bioengineering Institute, China; HL and PL test kits, purchased from Jianglai Biotechnology Co., Ltd., China; experimental reagents such as anhydrous ethanol, xylene, isopropanol neutral gum, purchased from Sinopharm Chemical Reagent Co., Ltd., China; HE dye solution, DAB color development agent, and oil red O dye solution, purchased from KeyGEN Biotechnology Co., Ltd, China; Antibodies IL-6 and TNF-α, purchased from Abcam limited.

Modeling scheme: 54 adult rats of 120±10 g were selected and fed adaptively for three days. They were then randomly divided to 6 groups, 9 animals for each group. They were blank control group, model group, positive control group, nuciferine group, and nuciferine structural modification group. The blank control group was fed with normal diet. The model group, positive group, nuciferine group, and nuciferine structural modification group were fed with high-fat diet (HFD) (composed of 77.8% standard diet, 10% protein powder, 10% lard, 2% cholesterol, and 0.2% bile salt, in which, contents of each index were as follows: lipid 41.5%, carbohydrate 40.2%, protein 18.3%). The feeding and modeling time was 5 weeks. Within 4 weeks after modeling, continuous administration was performed: the blank control group and model group were given 0.2 mL of distilled water by gastric gavage; the positive control group was administrated 20 mg/(kg·d) of simvastatin; while the treatment group was administrated 20 mg/(kg·d) of nuciferine or 20 mg/(kg·d) of a derivative of nuciferine (compounds **6a-6f).**

Blood sample collection: 24 h after the last gastric gavage administration, eyeballs were taken out and blood was drawn. Whole blood was collected in an EP tube, and centrifuged at 3000 r/min and 4 °C for 10 min, and the supernatant was collected and stored at -20 °C for blood biochemical indicator test.

Liver tissue sample collection: the rats were killed by dislocation, and the liver was taken out immediately after death, which was observed by naked eye to find out whether there was abnormal color and lesions, and was then rinsed with ice normal saline. The left lobe of the liver was quickly cut into small pieces and fixed in 10% formaldehyde overnight for sectioning. The tissue of the right lobe of the liver was quickly wrapped in tin box and stored at -80 °C for later use.

Preparation of 10% liver homogenate: the liver wrapped in tin box and stored in the ultra-low temperature freezer at -80 °C was taken out, and quickly placed on an ice box. 0.2 g of liver tissue was accurately taken, and ice normal saline with a volume 9 times that of the liver issue was added thereto. 10% tissue homogenate was formed by grounding and mixing, and then centrifuged at 3000 r/min and 4 °C for 10 min. The supernatant was collected for later use.

Biochemical indicator determination: indicators AST, ALT, TC, TG, NEFA, Bun, Cr, CAT, GSH-Px, GSH, MDA, HL, PL were determined according to the instructions of the kits.

Liver histopathological observation: the liver tissue fixed with 10% formaldehyde was dehydrated, transparentized, impregnated in wax, embedded, sectioned, and stained with HE. Pathological examination was carried out under a 40x objective microscope. The specific steps were as follows: 1. Dewaxing and dehydrating paraffin sections: sections were impregnated in xylene I for 20 min, xylene II for 20 min, anhydrous ethanol I for 5 min, anhydrous ethanol II for 5 min, 75% alcohol for 5 min, and washed with distilled water. 2. Staining with hematoxylin: sections were immerged in a hematoxylin dye solution for 3-5 min, washed with distilled water, differentiated with differentiation liquid, washed with distilled water, returned blue, and rinsed with flowing water. 3. Staining with eosin: the sections were dehydrated with 85% and 95% gradient alcohol sequentially for 5 min each, and then stained with eosin dye for 5 min. 4. dehydrating and sealing: sections were impregnated in anhydrous ethanol I for 5 min, anhydrous ethanol II for 5 min, anhydrous ethanol III for 5 min, dimethyl I for 5 min, xylene II for 5 min, and then sealed with transparent neutral gum. 5. Microscopy, image acquisition and analysis were performed.

Staining with Oil red O: 1. drying frozen sections (with a thickness of 4-8 µm) at room temperature for 15-20 min; 2. incubating in 100% isopropanol for 5 min (to avoid bringing water to oil red O); 3. incubating in 0.5% oil red O solution for 7-8 min; 4. washing with 85% isopropanol solution for 3 min; 5. double dehydration and washing with water; 6. hematoxylin staining for 1-1.5 min; 7. double dehydration and washing with water; 8. sealing, microscopy, image acquisition and analysis.

Immunohistochemical method: 1. dewaxing paraffin sections to water: the sections were sequentially impregnated in xylene I for 15 min, xylene II for 15 min, xylene III for 15 min, anhydrous ethanol I for 5 min, anhydrous ethanol II for 5 min, 85% alcohol for 5 min, 75% alcohol for 5 min, and washed in distilled water; 2. antigen repair: tissue sections were placed in a repair box filled with citric acid antigen repair buffer (pH=6.0), the repair box was placed in a microwave oven, and antigen repair was then carried out in the microwave oven, during which, medium fire was adopted and maintained for 8 min until boiling; fire ceasing was maintained for 8 min but heat preservation was also performed; medium-low fire was adopted and maintain ed for 7 min; during which, excessive evaporation of buffer should be prevented and the fully dry slide should be avoided. After natural cooling, the slide was placed in PBS (pH=7.4) and washed by shaking on the decolorizing shaker for 3 times, 5 min each time. 3. Blocking endogenous peroxidase: sections were placed into 3% hydrogen peroxide solution, and incubated at room temperature away from light for 25 min; the slides were placed in PBS (pH-7.4), and washed by shaking on a decolorizing shaker for 3 times, 5 min each time. 4. Serum blocking: the tissue was uniformly covered with 3%BSA by adding dropwise 3%BSA in the histochemical circle and blocked at room temperature for 30 min (those with primary antibody derived from goat were blocked with rabbit serum, and those with primary antibody of other sources were blocked with BSA). 5. Adding primary antibody: the blocking solution was removed by gently shaking, and primary antibody was added dropwise onto the sections, accompanied with primary antibody at a certain proportion; sections were placed flatly in a wet box at 4 °C and incubated therein overnight, (a small amount of water was added in the wet box to prevent the evaporation of antibody). 6. Adding secondary antibody: the slide was placed in PBS (pH=7.4) and washed by shaking on the decolorizing shaker for 3 times, 5 min each time; after the section was slightly dried, the tissue was covered with secondary antibody (labeled with HRP) of the corresponding species of primary antibody by adding the secondary antibody to the circle, and incubated at room temperature for 50 min. 7. DAB color development: the slide was placed in PBS (pH=7.4) and washed by shaking on a decolorizing shaker for 3 times, 5 min each time; after the section was slightly dried, the freshly prepared DAB color developing solution was added into the circle; the color developing time was controlled under the microscope, and brown and yellow was exhibited when it was positive, and the color development was terminated by rinsing the section with distilled water. 8. Restaining nucleus: nucleus was restained with hematoxylin for about 3 min, washed with distilled water, then differentiated with a hematoxylin differentiation solution for a few seconds, washed with distilled water, returned to blue with hematoxylin blue return solution, and washed with flowing water. 9. Dehydrating and sealing: the section was immerged in 75% alcohol for 5 min, 85% alcohol for 5 min, anhydrous ethanol I for 5 min, anhydrous ethanol II for 5 min, n-butanol for 5 min, xylene I for 5 min, dehydrated and transparentized; the section was taken out of xylene, dried slightly, and sealed with neutral gum. 10. Microscopy, image acquisition and analysis were performed.

### Results and discussion

### 2.1 Effect of nuciferine and its derivatives on liver and kidney indexes in hyperlipidemia rats

The liver and kidney indexes of hyperlipidemia rats induced by nuciferine and its derivatives are shown in Table 3. The results in Table 3 show that compared with the blank group, the liver and kidney indexes of rats in the model group increased significantly; the positive group and the drug to be tested groups significantly respond to this change to varying degrees, indicating that the drug to be tested groups have a corresponding protective effect on the liver and kidney of hyperlipidemia rats.

**Table 3 Effects of nuciferine and its derivatives on Liver Index in hyperlipidemia rats (x ± s, n=9)**

| Group | Liver index (%) | Kidney index (%) |
|---|---|---|
| Blank group | 3.17±0.24 | 0.65±0.02 |
| Model group | 5.03±0.27^{###} | 0.75±0.03^{###} |
| Simvastatin | 4.47±0.36** | 0.68±0.03** |
| Nuciferine | 4.52±0.30** | 0.68±0.04* |
| Compound **6a** | 4.58±0.29** | 0.68±0.04** |
| Compound **6b** | 4.58±0.23** | 0.68±0.02*** |
| Compound **6c** | 4.58±0.26** | 0.68±0.03*** |
| Compound **6d** | 4.58±0.24** | 0.68±0.04*** |
| Compound **6e** | 4.58±0.27** | 0.68±0.05*** |
| Compound **6f** | 4.58±0.25** | 0.68±0.03*** |

| | | |
|---|---|---|
| Note: Compared with the blank group, ### means P<0.001; compared with the model group, * means *P<0.05,* ** means P<0.01, *** means *P<0.001.* | | |

### 2.2 Effect of nuciferine and its derivatives on AST and ALT in hyperlipidemia rats

AST and ALT are commonly used indicators for liver function tests, and corresponding test results are shown in Table 4. As can be seen from Table 4, compared with the blank group, the serum AST and ALT contents of rats in the high-fat model group significantly increase. Compared with the model group, the drugs to be tested (i.e., nuciferine and its derivatives) significantly reduce the serum AST and ALT contents to varying degrees, among which, nuciferine has the best effect, and the effect is similar to that of the positive control group.

**Table 4 Effect of nuciferine and its derivatives on serum ALT and AST in hyperlipidemia rats (x ± s, n=9).**

| Experimental group | ALT (U/L) | AST (U/L) |
|---|---|---|
| Blank group | 4.20±1.67 | 5.40±1.60 |
| Model group | 29.43±10.22^{###} | 12.14±3.01^{##} |
| Simvastatin | 13.74±4.06** | 7.57±1.78** |
| Nuciferine | 13.97±5.71** | 6.43±1.56** |
| Compound **6a** | 15.46±8.79* | 7.85±2.23* |
| Compound **6b** | 16.43±9.51* | 7.91±2.21* |
| Compound **6c** | 16.44±8.69* | 7.86±2.24* |
| Compound **6d** | 15.62±9.43* | 7.78±2.27* |
| Compound **6e** | 15.54±8.69* | 7.65±2.26* |
| Compound **6f** | 16.73±9.74* | 7.74±2.22* |

| | | |
|---|---|---|
| Note: Compared with the blank group, ## means P<0.01, ### means P<0.001; compared with the model group, * means *P<0.05,* ** means *P*<0.01. | | |

### 2.3 Effect of nuciferine and its derivatives on blood lipids in hyperlipidemia rats

TC, TG, LDL-C, HDL-C are routine blood lipid evaluation indexes, and the corresponding test results are shown in Table 5. As can be seen from Table 5, compared with the blank group, serum TC, TG and LDL-C of rats in the model group significantly increase, while HDL-C is significantly decreased. Compared with the model group, the drugs to be tested (i.e., nuciferine and its derivatives) significantly reduce the contents of TC, TG and LDL-C in the serum of rats to varying degrees, and nuciferine significantly increases the content of HDL-C in the serum, while nuciferine derivative groups slightly increase the content of HDL-C in the serum, but the results do not have a significant effect.

**Table 5 Effect of nuciferine and its derivatives on blood lipids in hyperlipidemia rats**

| Experimental group | TC (mmol/L) | TG (mmol/L) | LDL-C (mmol/L) | HDL-C (mmol/L) |
|---|---|---|---|---|
| Blank group | 1.52±0.20 | 0.45±0.08 | 0.49±0.06 | 6.08±1.28 |
| Model group | 2.33±0.19^{###} | 0.68±0.04^{###} | 0.91±0.14^{##} | 4.36±0.53^{##} |
| Simvastatin | 2.00±0.19* | 0.47±0.05*** | 0.62±0.17* | 5.43±0.76** |
| Nuciferine | 1.90±0.19** | 0.48±0.04*** | 0.53±0.12*** | 5.32±0.53** |
| Compound **6a** | 1.94±0.18** | 0.56±0.05*** | 0.54±0.09*** | 4.85±0.73 |
| Compound **6b** | 2.01±0.14* | 0.57±0.08** | 0.68±0.16* | 4.90±0.92 |
| Compound **6c** | 1.93±0.16** | 0.56±0.07** | 0.54±0.08* | 4.85±0.72 |
| Compound **6d** | 2.03±0.09* | 0.57±0.07*** | 0.68±0.15*** | 4.90±0.87 |
| Compound **6e** | 1.95±0.13** | 0.55±0.05** | 0.56±0.08*** | 4.87±0.77 |
| Compound **6f** | 2.02±0.16* | 0.58±0.07** | 0.69±0.16* | 4.93±0.91 |

| | | | | |
|---|---|---|---|---|
| Note: Compared with the blank group, ## means *P*<0.01, ### means *P*<0.001; compared with the model group, * means *P<0.05,* ** means *P*<0.01, *** means *P<0.001.* | | | | |

### 2.4 Effect of nuciferine and its derivatives on Bun and Cr in hyperlipidemia rats

Bun and Cr are commonly used to evaluate renal function, and the corresponding test results are shown in Table 6. According to Table 6, Bun and Cr contents in the model group decrease significantly compared with those in the blank group. Compared with the model group, the positive group, the nuciferine group, and the nuciferine derivative group reduce Bun and Cr contents to varying degrees, and the results indicate having significant effects, and the test results are similar to that of the blank group, indicating that the kidney function tends to be normal.

**Table 6 Effects of nuciferine and its derivatives on serum Bun and Cr in hyperlipidemia rats (x ± s, n=9).**

| Experimental group | Bun(mmol/L) | Cr(µmol/L) |
|---|---|---|
| Blank group | 3.64 ± 0.25 | 48.72 ± 5.19 |
| Model group | 5.96 ± 0.74^{###} | 65.40 ± 7.86^{###} |
| Simvastatin | 3.98 ± 0.43*** | 53.79 ± 7.58** |
| Nuciferine | 3.27 ± 0.22*** | 52.59 ± 4.81** |
| Compound **6a** | 3.16 ± 0.17*** | 51.82 ± 7.06* |
| Compound **6b** | 3.48 ± 0.24*** | 53.90 ± 1.81** |
| Compound **6c** | 3.45 ± 0.51*** | 51.39 ± 5.83** |
| Compound **6d** | 3.56 ± 0.19*** | 53.82 ± 6.13* |
| Compound **6e** | 3.43 ± 0.27*** | 52.90 ± 2.21** |
| Compound **6f** | 3.62 ± 0.15*** | 52.82 ± 4.36* |

| | | |
|---|---|---|
| Note: Compared with the blank group, ### means P<0.001; compared with the model group, * means *P<0.05,* ** means *P*<0.01, *** means *P<0.001* | | |

### 2.5 Effects of nuciferine and its derivatives on liver TG and NEFA in hyperlipidemia rats

On the basis of the blood lipid levels, the expression levels of TG and NEFA in rat liver tissues were further determined. The results are shown in Table 7. As can be seen from Table 7, TG content and NEFA content significantly increase in the model group compared with the blank group. Compared with model group, TG contents in the positive group, the nuciferine group, and the nuciferine derivative group significantly decrease. NEFA content significantly decreases only in the nuciferine group, and no significant difference is found in other groups compared with the model group.

**Table 7 Effects of nuciferine and its derivatives on TG and NEFA in liver tissue of hyperlipidemia rats (x ± s, n=9).**

| Experimental group | TG (mmol/g prot) | NEFA (µmol/g prot) |
|---|---|---|
| Blank group | 0.06 ± 0.01 | 79.11 ± 24.83 |
| Model group | 0.24 ± 0.06^{###} | 118.20 ± 12.04^{#} |
| Simvastatin | 0.09 ± 0.01*** | 103.22 ± 8.89 |
| Nuciferine | 0.08 ± 0.01*** | 99.87 ± 8.25* |
| Compound **6a** | 0.10 ± 0.02*** | 107.69 ± 6.44 |
| Compound **6b** | 0.13 ± 0.02*** | 106.32 ± 22.95 |
| Compound **6c** | 0.10 ± 0.02*** | 107.69 ± 6.44 |
| Compound **6d** | 0.13 ± 0.02*** | 106.32 ± 22.95 |
| Compound **6e** | 0.10 ± 0.02*** | 107.69 ± 6.44 |
| Compound **6f** | 0.13 ± 0.02*** | 106.32 ± 22.95 |

| | | |
|---|---|---|
| Note: Compared with the blank group, # means *P*<0.05, ### means *P*<0.001; compared with the model group, * means *P<0.05,* ** means *P*<0.01, *** means *P<0.001.* | | |

### 2.6 Effect of nuciferine and its derivatives on the antioxidant capacity of liver tissue in hyperlipidemia rats

In the experiment, CAT, GSH-Px, GSH, and MDA were selected as indicators to evaluate effects of drugs to be tested on the antioxidant capacity of the liver tissue in hyperlipidemia rats. The results are shown in Table 8. As can be seen from Table 8, compared with the blank group, CAT, GSH-Px, and GSH contents significantly reduce in the model group, while MDA contents significantly increase, indicating a decrease in antioxidant enzymes and antioxidant substances and an increase in lipid peroxidation products in the liver tissue. It shows that hyperlipidemia rats are accompanied by dysregulation of antioxidant capacity in the liver tissue. Compared with the model group, tCAT and GSH contents in the positive group, the nuciferine group, and the nuciferine derivatives group significantly increase, the GSH-Px contents in the simvastatin group and the nuciferine derivatives group significantly increase, while the GSH-Px contents in the nuciferine group and the nuciferine derivative group do not significantly change. The MDA contents in the positive group, the nuciferine group, and the nuciferine derivative group significantly decrease. It indicates that nuciferine and its derivatives could enhance the antioxidant capacity of liver tissue with hyperlipidemia.

**Table 8 Effects of nuciferine and its modifications on the antioxidant capacity of liver tissue in hyperlipidemia rats (x ± s, n=9)**

| Experimental group | CAT (U/mgprot) | GSH-Px (U/mgprot) | GSH (µmol/gprot) | MDA (nmol/mgprot) |
|---|---|---|---|---|
| Blank group | 40.02 ± 12.38 | 780.02 ± 70.64 | 386.71 ± 62.58 | 0.71 ± 0.24 |
| Model group | 22.47 ± 3.54^{##} | 580.23 ± 59.18^{###} | 199.92 ± 26.75^{###} | 1.59 ± 0.51^{##} |
| Simvastatin | 29.35 ± 7.00* | 708.02 ± 47.00** | 341.36 ± 98.66*** | 0.84 ± 0.19** |
| Nuciferine | 28.44 ± 3.34** | 720.65 ± 59.57** | 377.68 ± 137.77** | 0.92 ± 0.15** |
| Compound **6a** | 26.06 ± 2.69* | 550.96 ± 60.97 | 360.02 ± 43.63*** | 0.94 ± 0.27* |
| Compound **6b** | 25.90 ± 4.29* | 532.14 ± 30.92 | 365.04 ± 71.78*** | 1.03 ± 0.33* |
| Compound **6c** | 27.43 ± 3.14** | 567.63 ± 51.57** | 374.63 ± 137.14** | 0.94 ± 0.38** |
| Compound **6d** | 25.78 ± 2.67* | 562.93 ± 68.91 | 360.02 ± 43.63*** | 0.95 ± 0.48* |
| Compound **6e** | 26.34 ± 4.54* | 537.18 ± 34.93 | 363.09± 71.72*** | 1.02 ± 0.73* |
| Compound **6f** | 27.42 ± 2.82* | 560.90 ± 62.45 | 361.04 ± 43.65*** | 0.98 ± 0.56* |

| | | | | |
|---|---|---|---|---|
| Note: Compared with the blank group, ## means *P*<0.01, ### means *P*<0.001; compared with the model group, * means *P<0.05,* ** means *P*<0.01, *** means *P<0.001.* | | | | |

### 2.7 Effect of nuciferine and its derivatives on HL and LPL in liver tissue in hyperlipidemia rats

HL and LPL are important fat metabolizing enzymes in the liver. The effects of drugs to be tested on the lipases HL and LPL in the liver tissue in hyperlipidemia rats were evaluated experimentally. The results are shown in Table 9. According to Table 9, compared with the blank group, lipase HL and LPL contents in the model group significantly reduce, indicating that the lipid metabolism capacity of liver tissue is weakened. Compared with the model group, the HL and LPL contents in liver tissue are significantly increased in the positive group, the nuciferine group, and the nuciferine derivative group, among which, the positive group and the nuciferine group have the best effect.

**Table 9 Effects of nuciferine and its derivatives on HL and LPL in liver issue in hyperlipidemia rats (x ± s, n=9)**

| Experimental group | HL (U/mgprot) | LPL (U/mgprot) |
|---|---|---|
| Blank group | 0.45 ± 0.05 | 0.53 ± 0.11 |
| Model group | 0.29 ± 0.05^{###} | 0.17 ± 0.04^{###} |
| Simvastatin | 0.45 ± 0.11** | 0.50 ± 0.06*** |
| Nuciferine | 0.47 ± 0.05*** | 0.43 ± 0.07*** |
| Compound **6a** | 0.35 ± 0.03* | 0.27 ± 0.03*** |
| Compound **6b** | 0.51 ± 0.05*** | 0.29 ± 0.10** |
| Compound **6c** | 0.44 ± 0.78** | 0.50 ± 0.07*** |
| Compound **6d** | 0.37 ± 0.08*** | 0.42 ± 0.06*** |
| Compound **6e** | 0.37 ± 0.07* | 0.28 ± 0.04*** |
| Compound **6f** | 0.56 ± 0.06*** | 0.26 ± 0.08** |

| | | |
|---|---|---|
| Note: Compared with the blank group, ### means *P*<0.001; compared with the model group, * means *P<0.05,* ** means *P*<0.01, *** means *P<0.001* | | |

### 2.8 Effect of nuciferine and its derivatives on pathological changes of liver in hyperlipidemia rats

FIG. 8 shows photographs of liver tissues in hyperlipidemia rats, in which, panel A represents blank group; panel B represents model group; panel C represents positive group; panel D represents nuciferine group; panel E represents benzoylnuciferine group; panel F represents p-fluorobenzoylnuciferine group. As can be seen from FIG. 8, the rat liver in the blank group is dark red, soft and elastic with sharp edges. Compared with the blank group, the rat liver in hyperlipidemia model group is slightly enlarged with blunt and thick edges, brittle, and shows greasy feeling when touching; and its color is obviously yellowish and whiter. Compared with the model group, the liver appearances in the simvastatin group and the drug tobe tested group are improved to different degrees: the liver is red, soft and elastic, with sharper edges. In contrast, the livers in the benzoylnuciferine group and the p-fluorobenzoylnuciferine group are more brittle.

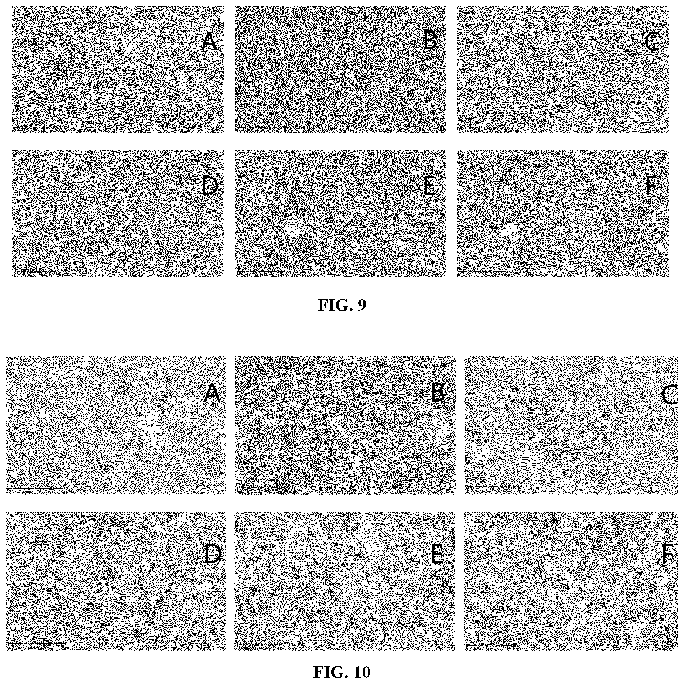

FIG. 9 shows the results of HE-stained liver tissues of hyperlipidemia rats, in which, panel A represents blank group; panel B represents model group; panel C represents positive group; panel D represents nuciferine group; panel E represents benzoylnuciferine group; panel F represents p-fluorobenzoylnuciferine group, all with a scale of 250 µm. The results in FIG. 9 show that, the liver lobule structure of rats in the blank group is complete, the liver cord is neatly arranged, and nuclei are centered, round and clear. Compared with the blank group, the liver lobule structure of rats in the model group is complete, the liver cord is more neatly arranged, the nucleus is clear, a large number of lipid vacuoles are present in the cell, some nuclei are obviously squeezed away from the centre position, and there is obvious inflammatory infiltration at the periphery of the blood vessels. In addition, some pathological sections show the pathological morphology of bulla steatosis, which are not shown. Compared with the model group, the liver lobule structure is intact, the liver cord is arranged neatly, the lipid vacuoles in hepatocytes are significantly reduced, and the inflammatory infiltration is alleviated in the nuciferine group and the nuciferine derivative group, showing that the drugs to be tested (i.e., nuciferine and its derivatives) could improve the adiposopathy and inflammatory infiltration in the liver of hyperlipidemia rats.

### 2.9 Nuciferine and its derivatives on oil red staining of liver in hyperlipidemia rats

FIG. 10 shows results of oil red O-stained liver tissues of hyperlipidemia rats, in which, panel A represents blank group; panel B represents model group; panel C represents positive group; panel D represents nuciferine group; panel E represents benzoylnuciferine group; panel F represents p-fluorobenzoylnuciferine group, all with a scale of 250 µm. As shown in FIG. 10, oil red stained liver sections of rats in the blank group is extremely shallow, indicating no significant lipid deposition. The oil red stained liver sections of rats in the hyperlipidemia model group is obvious, while the oil red stained liver sections in the drug to be tested groups (i.e., the nuciferine group and the nuciferine derivative group) is between the blank group and the model group, and shows a lighter color, indicating reduced fat accumulation. The results indicate that nuciferine and its derivatives could effectively reduce the accumulation of lipid in the liver of hyperlipidemia rats.

### 2.10 Effect of nuciferine and its derivatives on the expression of IL-6 and TNF-α in liver of hyperlipidemia rats

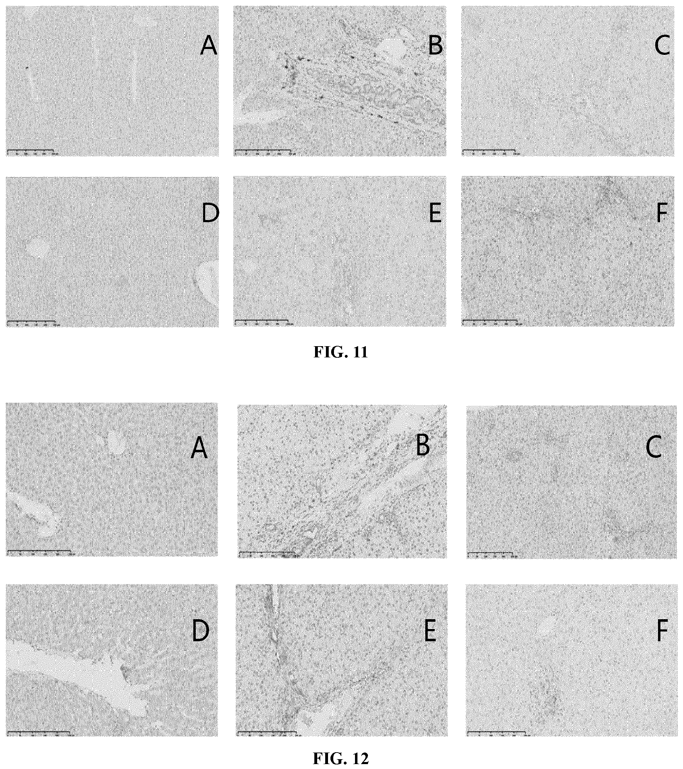

The expression levels of IL-6 and TNF-α inflammatory factors in the liver of hyperlipidemia rats were determined by immunohistochemistry tests. The results are shown in FIG. 11 and FIG. 12. FIG. 11 shows the expression of IL-6 in the liver tissue of hyperlipidemia rats. FIG. 12 shows the expression of TNF-α in the liver tissue of hyperlipidemia rats. In FIG. 11 and FIG. 12, panels A represent blank group; panels B represent model group; panels C represent positive group; panels D represent nuciferine group; panels E represent benzoylnuciferine group; and panels F represent p-fluorobenzoylnuciferine group, all with a scale of 250 µm. As shown in FIG. 11 and FIG. 12, the expression levels of IL-6 and TNF-α in the liver of rats in the blank group are lower, and the expression levels of IL-6 and TNF-α in the liver of rats in the hyperlipidemia model group significantly increase compared with the blank group. Compared with the model group, the expression levels of IL-6 and TNF-α decrease in the nuciferine group and the nuciferine derivative group. Among them, the effect of nuciferine is better than those of the two derivatives, showing that nuciferine could reduce the inflammation level in the liver of hyperlipidemia rats.

All of the above are only the preferred embodiments of the disclosure, and it should be noted that for technicians in the art, several improvements and refinements could be made without deviating from the principle of the disclosure, and these improvements and refinements shall also be deemed as being within the scope of the disclosure.

## Claims

1. A method for synthesizing nuciferine or a derivative thereof, comprising the steps of
(1) mixing a compound having a structure shown in formula IV, R-X, and an alkaline catalyst, and subjecting a resulting mixture to Schotten-Baumann reaction, to obtain a compound having a structure shown in formula V, wherein in the R-X, R represents aliphatic hydrocarbyl, aromatic hydrocarbyl, or acyl, and X represents halogen; and 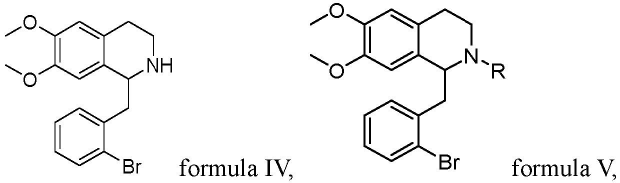
(2) mixing the compound having the structure shown in formula V, a phosphine ligand compound, an alkaline reagent, and a catalyst, and subjecting a resulting mixture to carbon-hydrogen bond activation reaction, to obtain the nuciferine or the derivative thereof, wherein
the nuciferine or the derivative thereof has a structure shown in formula VI, and 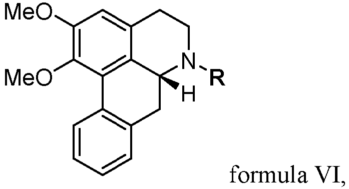
under the condition that R represents methyl, the compound shown in formula VI is the nuciferine, and under the condition that R represents other group, the compound shown in formula VI is the derivative of nuciferine.

2. The method as claimed in claim 1, wherein the alkaline catalyst in step (1) comprises at least one selected from the group consisting of an alkali metal hydroxide, an alkali metal carbonate, an alkali metal alkoxide, an amine compound, an ammonium salt, and an aminopyridine compound; and
a molar ratio of the compound having the structure shown in formula IV to the alkaline catalyst ranges from 1 : 1 to 1 : 2.

3. The method as claimed in claim 1, wherein a molar ratio of the compound having the structure shown in formula IV to the R-X ranges from 1 : 1 to 1 : 3.

4. The method as claimed in any one of claims 1 to 3, wherein the Schotten-Baumann reaction is performed at a temperature of 25-40 °C for 2-5 h.

5. The method as claimed in claim 1, wherein the alkaline reagent in step (2) comprises at least one selected from the group consisting of an alkali metal carbonate, an alkali metal bicarbonate, and an alkali metal hydroxide; and
a molar ratio of the compound having the structure shown in formula V to the alkaline reagent ranges from 1 : 2 to 1 : 4.

6. The method as claimed in claim 1, wherein the catalyst in step (2) comprises at least one selected from the group consisting of nickel chloride, bis(triphenylphosphine)nickel chloride, palladium chloride, tetra(triphenylphosphine)palladium, palladium acetate, and palladium on carbon; and
an amount in moles of the catalyst is 3-10% of an amount in moles of the compound having the structure shown in formula V

7. The method as claimed in claim 1, wherein an amount in moles of the phosphine ligand compound is 10-30% of an amount in moles of the compound having the structure shown in formula V

8. The method as claimed in claim 1 or 7, wherein the phosphine ligand compound comprises at least one selected from the group consisting of triphenylphosphine, tributylphosphine, tri-t-butylphosphine, tri-(o-methylphenyl)phosphine, tri-(p-tolyl)phosphine, and tri-(m-tolyl)phosphine.

9. The method as claimed in claim 1, wherein the carbon-hydrogen bond activation reaction is performed at a temperature of 120-150 °C for 8-15 h.

10. The method as claimed in claim 1, wherein the compound having the structure shown in formula V is prepared by a process comprising the following steps:
(i) mixing 2-bromophenylacetic acid, an acyl chlorination reagent, and a catalyst, subjecting a resulting mixture to acyl chlorination reaction, to obtain a compound having a structure shown in formula I; 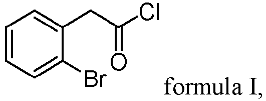
(ii) mixing the compound having the structure shown in formula I, 3,4-dimethoxyphenethylamine, and an alkaline reagent, and subjecting a resulting mixture to nucleophilic substitution reaction, to obtain a compound having a structure shown in formula II; 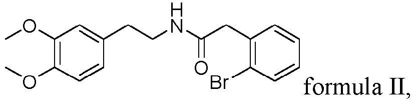
(iii) mixing the compound having the structure shown in formula II and POCl₃, and subjecting a resulting mixture to Bischler-Napieralski reaction, to obtain a compound having a structure shown in formula III; and 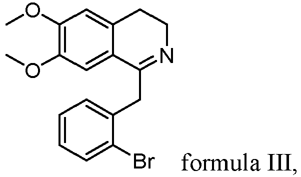
(iv) mixing the compound having the structure shown in formula III with a reducing agent, and subjecting a resulting mixture to reduction reaction, to obtain the compound having the structure shown in formula IV

11. The method as claimed in claim 10, wherein the acyl chlorination reagent in step (i) comprises at least one selected from the group consisting of phosphorus trichloride, phosphorus pentachloride, sulfoxide chloride, oxalyl chloride, and triphosgene; and
a molar ratio of the 2-bromophenylacetic acid to the acyl chlorination reagent ranges from 1 : 1 to 1 : 3.

12. The method as claimed in claim 10, wherein the catalyst in step (i) comprises at least one selected from the group consisting of N,N-dimethylformamide, ferric chloride, imidazole, and aluminum chloride; and
the catalyst is in an amount of 5% to 20% of a weight of the 2-bromophenylacetic acid .

13. The method as claimed in claim 10 or 12, wherein the acyl chlorination reaction is performed at a temperature of 20-50 °C for 2-5 h.

14. The method as claimed in claim 10, wherein the alkaline reagent in step (ii) comprises at least one selected from the group consisting of an alkali metal carbonate, an alkali metal hydroxide, triethylamine, and pyridine; and
a molar ratio of the alkaline reagent to the 3,4-dimethoxyphenethylamine ranges from 1 : 1 to 3 : 1.

15. The method as claimed in claim 10 or 14, wherein a molar ratio of the 3,4-dimethoxyphenethylamine to the compound having the structure shown in formula I ranges from 1 : 1 to 5 : 1.

16. The method as claimed in claim 10, wherein the nucleophilic substitution reaction is performed at a temperature of 25-40 °C for 8-15 h.

17. The method as claimed in claim 10, wherein in step (iii), a molar ratio of the compound having the structure shown in formula II to the POCl₃ ranges from 1 : 3 to 1 : 8.

18. The method as claimed in claim 10 or 17, wherein the Bischler-Napieralski reaction is performed at a temperature of 35-60 °C for 8-15 h.

19. The method as claimed in claim 10, wherein the reducing agent in step (iv) comprises at least one selected from the group consisting of sodium borohydride, potassium borohydride, lithium aluminum hydride, and borane; and
a molar ratio of the compound having the structure shown in formula III to the reducing agent ranges from 1 : 1 to 1 : 3.

20. The method as claimed in claim 10 or 19, wherein the reduction reaction is performed at a temperature of 20-40 °C for 2-5 h.

21. A derivative of nuciferine, having a structure shown in formula VI-1, 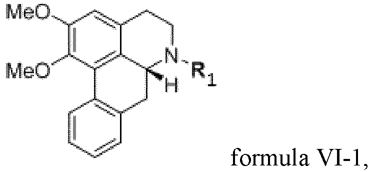 wherein in formula VI-1, R₁ represents non-methyl aliphatic hydrocarbyl, aromatic hydrocarbyl, or acyl.

22. The derivative of nuciferine as claimed in claim 21, wherein in formula VI-1, R₁ represents formyl, benzoyl, or a substituted benzoyl.

23. The derivative of nuciferine as claimed in claim 22, wherein the substituted benzoyl is one selected from the group consisting of p-fluorobenzoyl, p-methylbenzoyl, o-methylbenzoyl, and p-methoxybenzoyl.

24. Use of the derivative of nuciferine as claimed in any one of claims 21-23 in preparation of a lipid-lowering drug.

25. The use as claimed in claim 24, wherein the lipid-lowering drug is an antihyperlipidemic drug.

26. A method for treating hyperlipidemia, comprising
administrating the derivative of nuciferine as claimed in any one of claims 21 to 23.

27. The method as claimed in claim 26, wherein the derivative of nuciferine is administrated at a dose of 20 mg/ (kg·d).
